# EUROPEAN PATENT SPECIFICATION

(11) **EP 1 677 820 B1**
(45) Date of publication and mention of the grant of the patent: **04.04.2012**
(21) Application number: 04790870.2
(22) Date of filing: 15.10.2004
(51) Int. Cl.: A61K 39/00, C07K 14/47, C12N 15/12, C12N 5/10, C07K 16/30, G01N 33/574

(54) **COLORECTAL CANCER ANTIGEN**
KOLOREKTAL-KARZINOM-ANTIGEN
ANTIGENE DU CANCER COLORECTAL

(30) Priority: 15.10.2003 US 512040 P
(43) Date of publication of application: 12.07.2006
(73) Proprietor: ISTITUTO SUPERIORE DI SANITA', 00161 Roma (IT); NATIONAL INSTITUTES OF HEALTH, Rockville, MD 20852 (US)
(72) Inventor: ROBBINS, Paul, Bethesda, MD 20892 (US); ROSENBERG, Steven, Bethesda, MD 20892 (US); MACCALLI, Cristina, I-00161 Rome (IT)
(74) Representative: Icely, Dominic Michael
(86) International application number: PCT/EP2004/012087
(87) International publication number: WO 2005/039632

(56) References cited:
- WO-A-03/050253
- MACCALLI CRISTINA ET AL: "Identification of a colorectal tumor-associated antigen (COA-1) recognized by CD4+ T lymphocytes." CANCER RESEARCH, vol. 63, no. 20, 15 October 2003 (2003-10-15), pages 6735-6743, XP002319154 ISSN: 0008-5472
- SMITH CAROLINE L ET AL: "Immunotherapy of colorectal cancer." BRITISH MEDICAL BULLETIN. 2002, vol. 64, 2002, pages 181-200, XP008043772 ISSN: 0007-1420
- ZENG G: "MHC class II-restricted tumor antigens recognized by CD4+ T cells: new strategies for cancer vaccine design." JOURNAL OF IMMUNOTHERAPY (HAGERSTOWN, MD. : 1997) 2001 MAY-JUN, vol. 24, no. 3, May 2001 (2001-05), pages 195-204, XP008043810 ISSN: 1524-9557
- WANG RONG-FU: "Identification of MHC class II-restricted tumor antigens recognized by CD4+ T cells." METHODS (SAN DIEGO, CALIF.) MAR 2003, vol. 29, no. 3, March 2003 (2003-03), pages 227-235, XP002319744 ISSN: 1046-2023

## Description

The present invention relates to a novel, diagnostic antigen for colorectal cancer, uses thereof, and especially the use thereof in immunotherapeutic treatments for colorectal cancer.

### BACKGROUND OF THE INVENTION

Colon cancer is a leading cause of mortality in Western countries. Despite the improvement of surgery and chemotherapy treatments, the five-year survival rate has not significantly altered over several decades (1, 2). Immunological therapies have been intensively investigated in patients with melanoma, where treatment with IL-2, as well as the adoptive transfer of *in vitro* cultured tumour infiltrating lymphocytes (TIL), has been found to result in cancer regression in a significant percentage of patients (3, 4).

In contrast, immunotherapy has not provided a benefit to colorectal cancer patients, which may be due to the poor immunological characterization of this cancer, limiting the treatment options for patients with this disease (5, 6). The presence of a CD8⁺ T cell infiltrate in colon cancer has prognostic value (7); nevertheless, the presence of an inflammatory infiltrate was not linked to systemic immunity against cancer in this report. The loss of HLA class I expression both *in vitro* and *in vivo* has frequently been described in colorectal cancers, and appears to be associated with tumour progression (8-10).

The limited availability of *in vitro* established tumour lines and specific T lymphocytes has in addition hindered analysis of the role of the immune system in colorectal cancer. Although a large number of tumour associated antigens (TAA) have been identified, the majority of these are either limited in their expression to melanoma or are expressed in melanoma as well as in a number of other histologies, including breast, ovarian, lung and prostate tumours (11).

Candidate antigens that appear to be over-expressed in colon cancer, such as carcinoembryonic antigen (CEA), the epithelial cell adhesion molecule EP-CAM, HER-2/neu, and cyclophilin B, have been evaluated as potential targets for colorectal cancer therapy by carrying out *in vitro* sensitisations of PBMC with candidate peptides from these molecules that bind to particular HLA alleles.

However, only a relatively small number of potential epitopes have been identified, using this approach, and the T cells that have been generated, using many of these peptides, did not efficiently recognise native, unmanipulated tumour cells (12-15).

We have now identified a new tumour associated antigen for colorectal cancer that is capable of eliciting a T cell-mediated immune response. A colorectal antigen, termed COA-1, is known from Smith et al (Immunotherapy of colorectal cancer. British Medical Bulletin. 2002, vol. 64, 2002, pages 181-200) and WO 03/50253 (Incyte Genomics), but neither of these publications identify present SEQ ID No. 6, a specific epitopic fragment of COA-1, as being useful in a vaccine capable of obtaining T cells which efficiently recognise native, unmanipulated colorectal and melanoma cells.

### SUMMARY OF THE INVENTION

Thus, in a first aspect, the present invention provides a peptide, comprising all or an immunogenic part of the amino acid sequence designated SEQ ID NO 6, for use in a vaccine treatment of colorectal cancer or melanoma by stimulating an anti-colorectal cancer or an anti-melanoma immune response against COA-1 (SEQ ID NO 2), wherein the immunogenic part of the sequence is processed and expressed by antigen presenting cells in association with sympathetic MHC class II molecules.

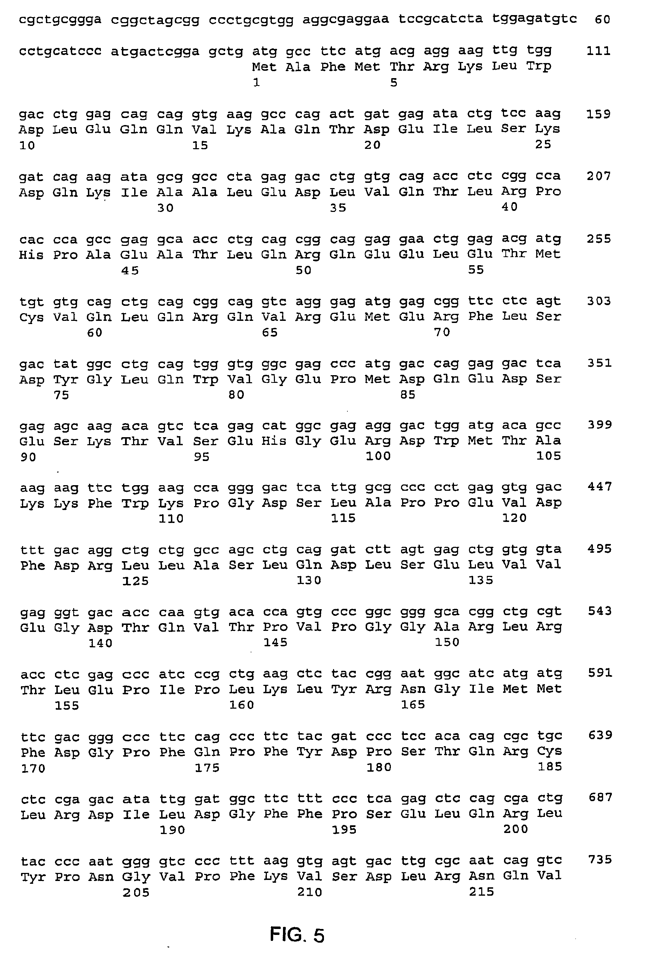

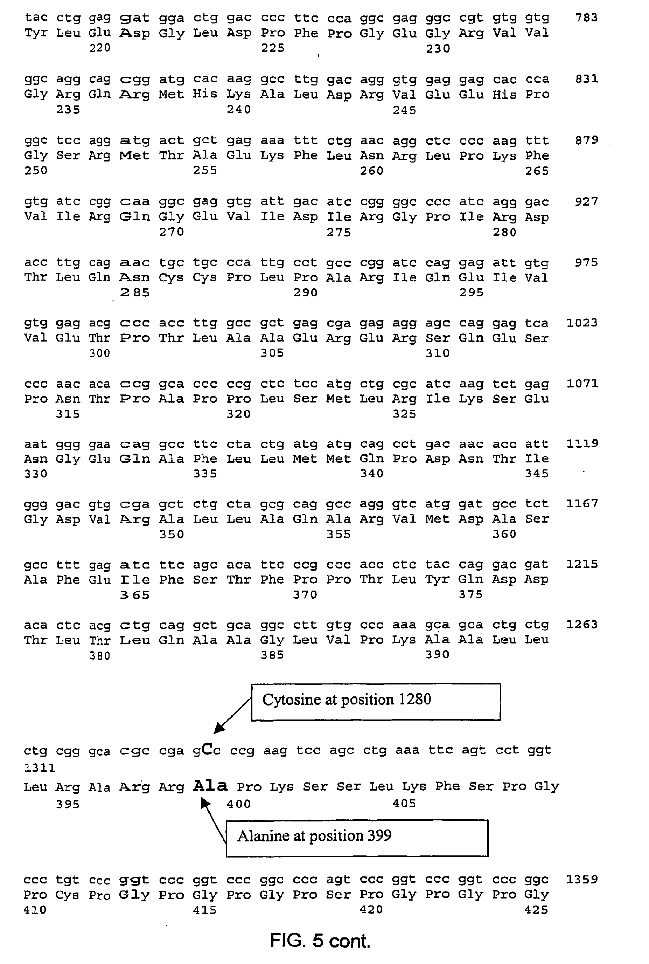

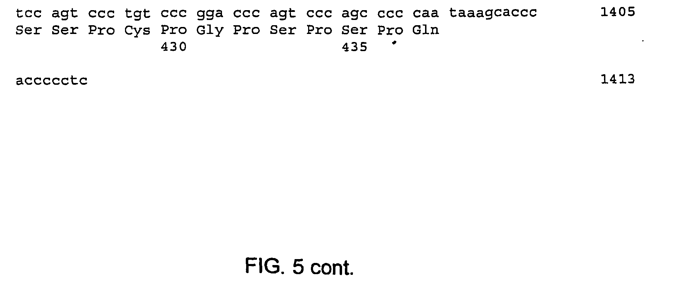

Also provided is said peptide for use in a method for stimulating immunity against colorectal cancer, comprising stimulating the production of antibodies against the human homologue of the Socius gene product, wherein the alanine residue at position 399 is substituted by a valine residue. The coding sequence, and the transcript thereof, for the colorectal antigen COA-1 are preferably as shown in SEQ ID NO 1, which shows the relationship of genetic sequence with the colorectal antigen COA-1 transcript (also shown in Figure 5), and which has alanine at position 399. It is SEQ ID No. 6, present within this larger sequence, which provides the antigen against which an immune reaction can be raised in accordance with the present invention.

The invention also provides the use of a peptide comprising all or an immunogenic part of the amino acid sequence designated SEQ ID NO 6 in the manufacture of a vaccine to stimulate an immune response against COA-1. The immunogenic part of the sequence is referred to herein as the epitopic portion of the sequence, and is sufficient to establish a response against COA-1, either as the isolated portion of the sequence, or in the context of any surrounding amino acid sequence(s) forming part of a longer sequence.

In particular, the immunogenic part of the sequence is sufficient, when administered in the form of a vaccine, to stimulate an immune response, particularly through the maturation of T cells.

The human homologue of the rat Socius gene product, as expressed in non-cancerous cells, also comprises alanine at the position corresponding to 399, although it has an extra 75 amino acid residues compared to the newly discovered COA-1 protein. The COA-1 protein has been shown to have either a valine or an alanine at position 399, the latter appearing to be associated with expression by cancerous cells, especially colorectal cancerous cells. Without being bound by theory, it appears that the presence of alanine at position 399 of the COA-1 protein is diagnostic, or at least indicative, of cancer in the tissue expressing it, at least where the tissue is colorectal.

The nucleotide sequence of the human homologue of the Socius gene and its gene product are shown in SEQ ID NOS. 19 and 20 respectively.

However, what is particularly surprising is that it has been established that an epitope located between amino acids 372 and 385, inclusive, of the COA-1 transcript is responsible for stimulating immunity against the tumour variant of the protein, and that it is not necessary for the immunising peptide to comprise the mutation at position 399.

The immunising peptide comprises an epitopic portion of the peptide TLYQDDTLTLQAAG (SEQ ID NO. 6). This sequence may be supplemented with additional sequences at either end, up to and including the entire remaining sequences of COA-1, and even additional sequences beyond that, if desired, such as might be encountered with a fusion protein, for example. As demonstrated herein, more specific supplemental sequences, including FSTFPP (SEQ ID NO. 9) at the N-terminus and/or LVPKAA (SEQ ID NO. 10) at the C-terminus both permit stimulation. It will be appreciated that, in general, an epitope need not be as long as 14 amino acids, and that a deletion of a few amino acid residues from either end of the epitope may still serve to produce immunity.

Thus, the present invention contemplates a peptide sequence comprising an epitopic portion of SEQ ID NO. 6. The epitopic portion preferably consists of 8 or more, and preferably 10 or more, contiguous amino acid residues from SEQ ID NO. 6. Where they are part of a longer peptide or other molecule, then the epitopic portion is preferably either suitably exposed to be able to stimulate an immune response, or is presented in such a manner as to be processable to achieve such stimulation when presented to the host's immune system. In this respect, it is generally not desirable to use full length COA-1 protein, mutated into the cancerous form, or otherwise, as the epitope can be cryptic, in this form.

It has also been established that the epitope is preferentially expressed by antigen presenting cells in association with the alleles HLA DRβ1*0402 or HLA DRβ1*1301. It will be appreciated that these sympathetic alleles are not necessarily the only HLA alleles able to stimulate immunity to COA-1, and that the present invention extends to other sympathetic alleles. Preferably the epitope is preferentially expressed by antigen presenting cells in association with either or both of the HLA DRβ1*0402 or HLA DRβ1*1301 alleles.

Sympathetic HLA-II alleles are not necessarily present in all members of the human population but, where an individual has PBMC's (peripheral blood mononuclear cells) either autologous or allogeneic for either of these alleles, then it is sufficient simply to provide a vaccine comprising the immunising part of COA-1.

The immunising portion of COA-1 may be as much as the entire molecule, either with or without the mutation at position 399 but, more preferably, it simply comprises a peptide comprising at least the immunising epitope located between position 372 and 385 of the COA-1 transcript. The invention further extends to the sequence between 371 and 384, inclusive, of COA-1 as an epitope, as well as to the sequence 371 to 385, inclusive, and 372 to 384, inclusive.

The immunising epitope may be presented in any suitable form. At its simplest, a vaccine comprising the peptide and a suitable carrier may be provided, together with, if required, any suitable excipients and/or adjuvants, for example.

The immunogenic peptide may also be presented in the form of nucleic acid in a form suitable for expression in the patient, either in a host organism, such as an attenuated virus, in a vaccine, or in the form of a suitable expression vector for expression *in vivo*.

It will be appreciated that the present invention extends to the sequence for COA-1, as well as the transcription product thereof. The invention further extends to the COA-1 sequence lacking one or more introns. The sequence of the invention may also lack one or more exons, provided that the immunising epitope provided between amino acids 372 and 385 of the wild type transcript is encoded. It is not necessary for the amino acid substitution at position 399 to be encoded, and it is generally preferred that this substitution is not encoded by the nucleotide sequences of the present invention. Without being bound by theory, it is possible that this substitution in the sequence of normal cells could affect the processing of the antigen, leading to a lack of expression of the immunogenic epitope. It will be appreciated that the degeneracy of the genetic code allows the nucleotide sequence to vary widely and still encode the immunogenic sequence, but it is generally preferred to use the wild-type sequence, for simplicity, unless it is desired to engineer a splice site, for example.

Where the patient does not express a sympathetic HLA-II allele, then immunity may be conferred in a number of ways, any of which may also be employed in patients expressing a sympathetic allele.

Sympathetic alleles are expressed by PBMC's, such as B cells and fibroblasts. Thus, in one aspect, it is sufficient to isolate PBMC's or their progenitors from the patient and to transform these cells with HLA DRβ1*1301 or HLA DRβ1*0402 alleles, for example. Once successful transformation has been achieved, then the PBMC's, whether directly transformed, or whether obtained from the progenitors, may be used to stimulate the appropriate immunity, after reintroduction into the patient. This may be achieved either by introducing the PBMC's into the patient, followed by administration of a vaccine as described above, or the PBMC's may be contacted with COA-1, or a precursor therefor, or the immunising epitope or precursor therefor and, preferably once there has been some opportunity, for endocytosis to occur, the treated PBMC's are administered to the patient. It will be appreciated that, in these circumstances, a "precursor" may include, for example, a fusion protein or a nucleic acid suitable for expression in the PBMC culture.

It will also be appreciated that suitable PBMC's may be obtained from, for example, a universal donor, and an immunising preparation may be made from such cells in a manner similar to that described above for transformed cells from the patients themselves.

It will be appreciated that the present invention extends to vaccines and immunising preparations as described above, as well as to host cells expressing COA-1, or a precursor therefor, provided that the immunising epitope is comprised in the transcript expressed thereby.

It will also be appreciated that the present invention extends to the use of antibodies recognising COA-1 having alanine at position 399. Such antibodies may be used as a passive vaccine, for example or may be used in diagnostic assays for colorectal cancer. Such assays may take the form of ELISA assays, for example, or may be used in suitable immunoblotting techniques.

The invention extends to the COA-1 protein, and especially to fragments thereof comprising an epitopic sequence, as defined above. Such fragments may further comprise additional amino acid residues up to and including alanine at position 399 of SEQ ID NO. 1, and includes such fragments where residues between the epitope and position 399 are conservatively substituted, or there are one or more deletions, insertions and/or inversions that do not block the antigenicity of the epitope.

The invention further provides a vaccine comprising a peptide of the invention and PBMC's expressing a sympathetic allele therefor, preferably an MHC Class II allele.

Thus, COA-1 is thought to be an immunodominant antigen mediating an anti-tumour immune response in Colorectal Cancer (CRC) patients. COA-1 is, therefore, thought to be useful as an immunogenic antigen for mediating an anti-tumour immune responses in CRC patients, the response preferably correlating with the progression of the disease. Thus, it is also thought to be useful in the provision of immunotherapy protocols, such as peptide vaccination or adoptive transfer of antigen specific T cells for CRC, as well as being a useful marker for the prognosis of the disease.

Preferably, the peptide is an oligopeptide, preferably having 50% or less of the amino acid sequence of COA-1, preferably 40% or less, preferably 30% or less, preferably 20% or less, and most preferably 10% or less.

Preferably, the peptide comprises the amino acid sequence designated SEQ ID NO 6, and raises an immunogenic response by administration thereof. Preferably, eliciting a CD4⁺ Tcell response in an individual.

We have also found that the peptide raises an immunogenic response in melanoma cells. Therefore, it is also preferred that the immune response is stimulated against melanoma cells.

### DESCRIPTION OF THE DRAWINGS

In the following Example reference is made to the accompanying Figures, in which:
**Figure 1** **shows a phenotypic characterization of the colorectal cancer line 1869 col.**
Figure 1A shows a stained 1869 col cell line using antibodies directed against MHC class I (W6/32) and class II (L243) molecules, an epithelium marker (Ber-EP4), and the β subunit of prolyl-4-hydroxylase (5B5), a protein expressed exclusively in fibroblasts.
Figure 1B shows intracellular staining carried out using three cytokeratin reactive monoclonal antibodies: CK18, which reacts with cytokeratin 18; LP34, which reacts with multiple cytokeratins; and MNF116, which reacts with cytokeratins 5, 6, 8, 17 and probably 19.
Figure 1C shows staining of 1869 col cells at passage 6 (P6) and passage 20 (P20), carried out with the anti-CEA monoclonal antibody Col-1.
**Figure 2** **shows a cDNA clone isolated from the 1869 cDNA library encoding an antigen recognised by C111 T cells.**
   The 293 cells expressing the MHC DRβ1*0402 or 1301 molecules were transfected with the 1D8 cDNA clone, or COA-1a, which corresponds to nucleotides 209-1318 of the COA-1 gene (see Figure 3).
   Target cells were either transfected with the COA-1a product alone or were co-transfected with a mixture of COA-1a and the full length HLA class II invariant chain (II). Additional targets were transfected with a control plasmid encoding GFP. Eighteen hours following the addition of 5x10⁴ C111 T cells to the transfectants, supernatants were collected and IFN-γ release was measured by ELISA.

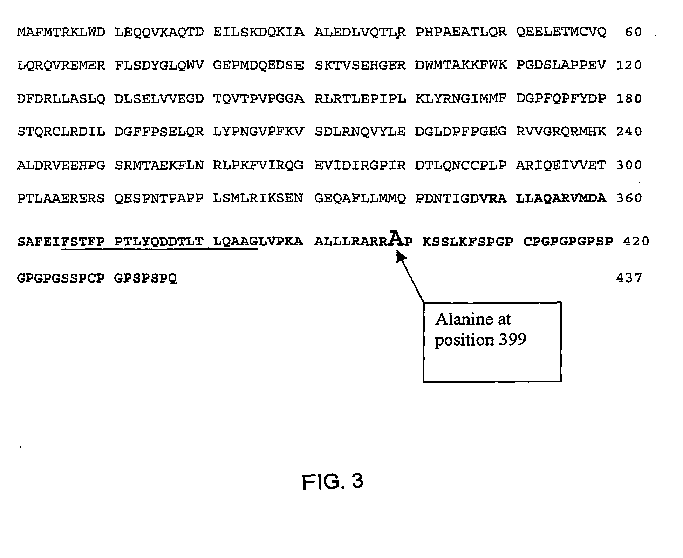

**Figure 3** **provides the sequence of the COA-1 gene (SEQ ID NO.1) isolated from the mRNA of the tumour line 1869 col.**
   The COA-1 gene was isolated by RT-PCR from the 1869 col tumour cell line. The amino acid sequence of the 1D8 cDNA clone (SEQ ID NO. 12) is shown in bold letters. The amino acid sequence corresponding to the T cell epitope (SEQ ID NO.6) is underlined, and the single nucleotide difference between the normal and tumour transcripts at position 1280 is noted.

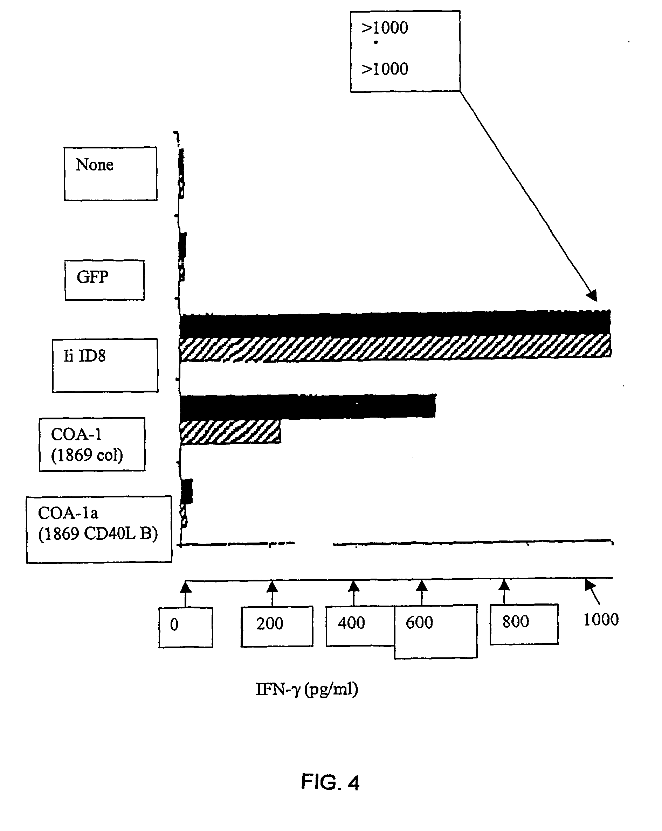

**Figure 4** **shows that the COA-1 transcript derived from normal B cells is not recognised by the clone C111 T cells.**
   293 cells expressing the indicated MHC DRβ1 molecules were transfected with COA-1a cDNAs isolated by RT-PCR from either the 1869 col cell line or from 1869 CD40L stimulated B cells. The GFP and Ii-1D8 constructs were used as negative and positive controls, respectively. Eighteen hours following the addition of 5x10⁴ C111 T cells to the transfectants, supernatants were collected and IFN-γ release was measured by ELISA. Dark shading represents 293-DR*1301. Hatched shading represents 23-DR*0402.
**Figure 5** **shows the relationship of the genetic sequence of COA-1 to the transcript.**
   The nucleotide sequence of COA-1 is shown in relationship to the protein sequence. The gCc triplet comprising C at nucleotide position 1280, encodes Alanine The amino acid sequence (SEQ ID NO. 2) of the longest open reading frame in this transcript, which is similar to the Socius gene product (20), is noted beneath the nucleotide sequence.

### DETAILED DESCRIPTION OF THE INVENTION

Several tumour reactive CD4⁺ T lymphocytes were isolated from PBMC and TIL that were obtained following the establishment of autologous cultured colon tumour cell lines. These studies focused on a single clone of CD4⁺ T cells, C111, that responded strongly to autologous tumour cells, and demonstrated low but significant reactivity with autologous EBV B cells, but failed to respond to autologous CD40L stimulated B cells. The gene encoding this antigen, termed COA-1, was isolated by screening an autologous cDNA library with clone C111 T cells. This gene appeared to be nearly identical to the gene encoding the human homologue of the rat Socius protein that was recently cloned using a yeast two-hybrid screening assay in which a member of the Rnd family of GTPases was used as bait (20). The Socius product was expressed at high levels in rat testis, but was expressed at significantly lower levels in rat lung, thymus and brain.

The longest open reading frame in the COA-1 transcript encodes a 437 amino acid product that corresponds to a portion of the human Socius gene product, and two overlapping peptides derived from this open reading frame were identified that could sensitise target cells expressing either HLA-DRβ1*0402 or 1301. The stimulation observed with peptide pulsed targets was weak relative to that seen with the tumour cell lines that were recognised, and a minimum concentration of approximately 10 µM was needed to stimulate significant cytokine release from C111 T cells (Table 4).

Peptides derived from non-mutated tumour antigens such as tyrosinase (23) and TRP-1 or TRP-2 (17) have also been found to stimulate only relatively low levels of cytokine release from HLA class II-restricted, tumour reactive T cells, and minimal concentrations of between 1 and 10 µM of the peptides identified in these studies were required to sensitise target cells for T cell recognition. This may reflect the fact that these represent non-mutated self antigens, and that self tolerance results in the deletion of T cells that recognise peptides that bind to class II molecules with high affinity.

In addition, the autologous tumour cell line should present this peptide in the context of both the HLA-DRβ1*0402 and 1301 restriction elements, leading to enhanced stimulation of T cells reactive with this epitope. Transfectants expressing the COA-1 product stimulated significantly less cytokine release from C111 T cells than the autologous tumour cell line that had been induced to express high levels of HLA class II molecules. One potential explanation for this observation, however, is that the HLA class II positive 293 cells used as targets for transfection of the COA-1 gene products fail to express optimal levels of accessory molecules associated with the processing of this epitope.

The COA-1 transcript is nearly identical to sequences derived from a variety of tissues and tumour cell lines. These transcripts, however, comprise a large array of over 20 alternatively spliced products that are derived from at least 15 exons residing at the chromosome 1p36.1-p35 locus. The COA-1 product expressed in colon tumour cell lines appeared to contain a unique splicing pattern that did not correspond to any of the transcripts identified in the EST and GenBank databases, which may not encode products recognised by C111 T cells. Two nearly identical COA-1 gene products were amplified from EBV B cells, one of which was identical to that isolated from the colon tumour cells, and a second that contained a single nucleotide alteration at position 1280 that resulted in a substitution of a valine residue for the alanine residue at position 399 encoded by the dominant colon tumour cell product. It is not clear why C111 T cells only appeared to weakly recognise EBV B cells expressing the appropriate HLA class II gene products, but these observations could result from inherent differences in the antigen processing abilities of colon tumour cells and EBV transformed B cells.

Previous results have suggested that differences in the proteosomal subunits expressed by various cells may significantly influence antigen recognition, which provides one potential explanation for this finding (24). The RT-PCR products that were amplified from normal B cells and fibroblasts also appeared to uniquely encode the COA-1 variant that expressed a valine residue at amino acid 399, and target cells that were transfected with the COA-1 product that was amplified from normal cell lines were not recognised by C111 T cells.

Thus, it appears that normal B cells and fibroblasts either fail to express the COA-1 transcript that can be processed and presented to C111 T cells or express this product at only relatively low levels. The mechanisms involved in the preferential expression of these two transcripts are unknown, but these may represent the products of two nearly identical genes whose expression is differentially regulated. The correlation between expression of these products and the ability of C111 T cells to recognise the epitope encoded by these products provides further evidence that this represents the natural product recognised by these T cells and not a peptide mimic of the natural epitope.

An additional observation, that is further discussed below, is how the alteration at position 399 affects recognition of the cell epitope comprised of amino acids 372 to 385 of the COA-1 transcript. Results of a previous study indicated that alteration of a distal residue can influence the ability of tumour reactive CD4+ T cells to recognise a mutated product of the CDC-27 gene product (21). Preliminary results presented in the prior study indicated that altered intracellular targeting of the mutated CDC-27 gene product may have played an important role in influencing processing of this gene product. Investigation of the cellular localization of the COA-1 protein in normal and tumour cells may help to indicate whether a similar mechanism may be involved with T cell recognition of this product.

Transfection studies, as well as peptide pulsing experiments, indicated that either of the autologous HLA-DRβ1 alleles, DRβ1*0402 or DRβ1*1301 could present the T cell epitope to clone C111 T cells, which may potentially enhance the immunogenicity of this peptide in patient 1869 as well as other individuals that express these class II alleles. This observation is not unique, however, as examples of promiscuous recognition of class II and well as class I restricted epitopes have been noted in previous studies. In one report, CD4+ T cells were identified that also recognised an epitope of the herpes simplex type 2 virus virion protein, VP16 in the context of DRβ1*0402, 1102 or 1301 but not several closely related DR4, 11 or 13 subtypes (25). The sequences of the DRβ1*0402, 1102 and 1301 molecules are identical in a polymorphic region between amino acids 67 and 71, and site directed mutagenesis studies demonstrated that these residues were critical for the recognition of the viral epitope.

High levels of lymphocyte infiltration into tumours have been shown in some studies to be correlated with a good prognosis (26), but detailed investigations of the reactivity of infiltrating T cells have not been carried out. The expression of HLA class II molecules on colorectal cancer cells is also a favourable prognostic marker (27) (28). Previous studies resulted in the isolation of HLA class I (29) and class II (30, 31) restricted tumour reactive T cells from colon cancer patients, but only a limited panel of shared tumour specific antigens were identified in these studies.

Peripheral blood lymphocytes isolated from CRC (colorectal cancer) patients were *in vitro* stimulated with the COA-1 derived epitope and tumour reactivity has been verified. Tumour-specific CD4⁺ T cells were isolated from 3 patients with progressive disease; although a single failure in generating COA-1 specific T cells was observed in CRC patient (n.4) with early stage tumour.

In collaboration with the clinical centre of the Fatebenefratelli Hospital, Rome, peripheral blood samples from CRC patients have been collected to confirm whether an immune response directed to COA-1 is commonly detectable in a large number of patients expressing specific MHC class II molecules and with metastatic disease. These results seek to demonstrate that COA-1 is a relevant antigen for the anti-tumour immune response in CRC patients correlating with the progression of the disease.

In addition, we have also shown that COA-1-specific reactivity could be isolated from PBMCs of CRC patients using professional antigen presenting cells, dendritic cells (DC) loaded with tumour expressed antigen array. DC were generated, in the presence of GM-CSF and IFN-alpfa, from monocytes of one CRC patient (anti-COA-1 T cells were previously isolated from the same patient, in the Example), loaded with autologous CRC line-derived lysate and used for *in vitro* stimulation of PBMCs.

After three stimulations both anti-COA-1 and tumour reactive T cells have been isolated. Tumour reactive and COA-1 specific CD4⁺ T cells could be isolated from the same CRC patient by *in vitro* stimulation of PBMCs either with intact tumour cells and with DC pulsed with tumour lysate. These results indicate that COA-1 can represent an immunodominant antigen mediating an anti-tumour immune response in CRC patients.

COA-1 specific T cells recognised specifically only tumour cells and not normal cells, though both types of cells express this antigen (see the Example), suggesting that a differential localization and/or processing of this antigen could occur in malignant or normal cells. To investigate this issue, a laser scanning confocal microscopy analysis was carried out on a panel of normal and tumour cell lines by using a specific polyclonal antibody directed to COA-1. The intra-cellular localisation and the translocation pathway to the cell membrane of COA-1 were studied.

Localisation of the protein in the cellular cytoplasm was observed both in tumour and in normal cells, whereas nuclear localization of the protein was found only in CRC and fibroblasts cell lines. Association of the protein with Golgi apparatus has been selectively detected in tumour cells and, moreover, co-loealization of COA-1 with one of the microtubule components, tubulin, occurred only in fibroblasts.

It is notable that COA-1 was only associated with HLA class II molecules in tumour cells. Thus, taken together these results indicate that, with regard to the COA-1 antigen, differential localisation and distinct pathways of cellular translocation occurred in normal and malignant cells.

Therefore, we conclude that the differential localisation of the protein could affect the HLA molecule-associated presentation of COA-1-derived immunogenic epitopes, resulting in the antigen's ability to raise a tumour specific immune response.

The recombinant COA-1 protein has now been synthesised, and this can be used to produce specific antibodies, including monoclonal antibodies. In addition, a multimeric immunogenic peptide, a complex ofmultiple chains of the COA-1-derived epitope, has been synthesized and used to produce antibodies specific for the epitope of COA-1 that can raise an immune response.

These reagents represent useful tools for evaluating the presence of antibodies directed to COA-1, or of the protein itself, in the serum of CRC patients. Moreover, this investigation can be correlated to the follow-up of patients to evaluate COA-1 as a prognostic marker for the disease. In addition, the new synthesized anti-COA-1 antibodies can be used to confirm the results of the analysis of COA-1 cellular localization.

The invention is not to be limited by what has been particularly shown and described, except as indicated by the appended claims. Indeed, the invention will now be illustrated in connection with the following Example.

### Example

### Material and Methods

### Cell lines and antibodies.

Colon cancer lines were generated from tumour liver metastases of five patients admitted to the Surgery Branch, National Cancer Institute, National Institutes of Health, Bethesda, MD, USA. The cell lines were generated from the tumour samples by cutting the tissue into small fragments, followed by filtration through sterile gauze. The tumour cells were cultured in collagen-coated 6-well plates (Becton Dickinson, Franklin Lakes, NJ) in ACL-4 medium (InVitrogen, Carlsbard, CA) containing 10 % foetal bovine serum plus MEGM SingleQuots (Clonetics, Walkersville, MD) that contained epidermal growth factor (10 ng /ml), insulin (5 µg/ml), hydrocortisone (0.5 µg /ml), gentamicin (50 µg/ml), and amphotericin-B (50 ng/ml). Fresh medium was added to the cells every 5 days and fibroblasts were depleted from the cultures by carrying out a short-term treatment with trypsin. Immunofluorescent staining assays to assess cell surface HLA gene expression were carried out using the anti-class I mAb W6/32 and the anti-DR mAb L243 (Becton Dickinson).

The cell lines were stained using the mAb BerEP4 (DAKO, Cupertino, CA) that is directed against a cell surface molecule whose expression appears to be limited to epithelial tissues, and intracellular staining was carried out using the cytokeratin reactive mAbs CD 18, LP34 and MNF116 (DAKO). Analysis of the expression of carcinoembryonic antigen (CEA), a molecule that is frequently over-expressed in colon tumour, was carried out using the mAb Col-1 (Zymed, South San Francisco, CA). The presence of fibroblasts in the cultured colon tumour cell lines was assessed using the mAb 5B5 (DAKO) that was directed against the β subunit of prolyl-4-hydroxylase, a protein involved with the synthesis of collagen. Flow cytometry was carried out using a FACScan (Becton Dickinson). The established colon cancer lines SW1463, SW480 and Colo205 were obtain from American Type Culture Collection (ATCC, Manassas, Virginia). The melanoma cell line 1681, the fibroblast cell line 1519 and the EBV-transformed B cell lines 1869 and 1519 were established in the Surgery Branch and were cultured in RPMI plus 10 % FBS. The normal B cell lines 1847, 1681, 1872 and 1869 were generated, as previously described (16), by culturing PBL in ISCOVE's medium (InVitrogen) plus 10 % human serum in the presence of 100 IU/ml of CD40L (Immunex, Seattle, WA) and 100 IU/ml of recombinant human IL-4 (Pharmingen, San Diego, CA). The MHC class I and class II typing of the PBL and of the tumour lines used in this study was determined by single-stranded oligonucleotide probe-PCR typing carried out in the NIH HLA typing laboratory, and is summarised in Table 1. Antibodies used to carry out T cell receptor (TCR) analysis were obtained from Beckman/Coulter (Miami, FL) or Pierce/Endogen (Rockford, II.).

### Identification and characterization of tumour reactive T cells.

Tumour reactive T lymphocytes were generated from PBMC and tumour infiltrating lymphocytes (TIL) derived from colon cancer patients. Incubation of PBMC with autologous tumour cells that had been irradiated with 150 Gy was carried out at a tumour cell to lymphocyte ratio of 1 to 5 in RPMI media containing 300 IU/ml of recombinant human IL-2 plus 10 % human serum (HS). The cultures were stimulated weekly for a period of 5 to 6 weeks with autologous irradiated tumour cells. Cultures of TIL were established by initially plating fresh uncultured tumours at 5x10⁵ cells per well in 24-well plates in RPMI containing 10 % HS and 1,000 IU/ml of IL-2. Tumour cells used for T cell stimulation were cultured for at least 10 days in RPMI containing 10 % HS to avoid the generation of T cells with reactivity against FBS. In addition, to optimise or up-regulate the expression of MHC molecules by tumour cells, these cells were incubated with IFN-γ (500 IU/ml) for 48 hr. The reactivity of the T cell lines against colon cancer lines was examined by incubation of 2X10⁴ or, for some of the assays, 5X10⁴ T cells in flat bottom 96-well plate in the presence of 5x10⁴ autologous or allogeneic tumour cells. After overnight incubation at 37°C in 5 % CO₂, the supernatants were collected and T cell responses were evaluated using anti-IFN-γ antibodies (Endogen, Rockford, IL) in a sandwich ELISA assay.

After 3 weeks of culture the T cell lines were cloned by limiting dilution in the presence of allogeneic PBMC that had been irradiated with 50 Gy in RPMI media containing 30 ng/ml of OKT3 mAb in RPMI plus 10 % HS. The following day, fresh medium plus rh-IL-2 (300 IU/ml) was added to the cultures. After two weeks of culture, growth positive wells were screened for their ability to release IFN-γ in response to tumour stimulation. The T lymphocytes from sensitised PBMC that were chosen for further analysis, C4, C49 and C111, were isolated from cultures that were plated at 5 cells per well, but only 27% of the wells were positive for growth under these conditions, showing that some or all of these cells represent T cell clones.

Analysis carried out with antibodies directed against T cell receptor (TCR) families showed that greater than 95% of clone C4 T cells expressed a TCR reactive with an anti-Vβ5 reactive antibody, whereas C49 failed to express TCRs detected by any of the commercial antibodies. Amplification of the clone C111 TCR Vβ region product carried out using RT-PCR showed that this clone expressed a single sequence derived from the Vβ18 germline gene. Flow cytofluorimetric analysis showed that approximately 80% of C111 T cells expressed Vb18, but contaminating feeder cells used to expand the T cell clone may be responsible for the discrepancy between these results. Two CD4+ tumour reactive T cell cultures, C5 and C15, were also identified from 1869 TIL. These cultures were isolated from cells that were plated at one cell per well, and, as only 3% of the wells that were plated were positive for growth, these represent T cell clones. In addition, these cultures stained homogeneously with an antibody directed against Vb2, further showing that these represented T cell clones.

Tumour reactive cultures were then expanded in the presence of allogeneic PBL that were irradiated with 50 Gy in RPMI containing PHA (1µg/ml) and IL-2 (300 IU/ml). Immunofluorescent analysis of positive cultures was carried out using mAb directed against CD3, CD4, CD8, CD16, and CD56 (Becton Dickinson). Antibody blocking assays were carried out by pre-incubating target cells for 1 hour with W6/32, an antibody directed against a pan-MHC class I epitope, or L243, a mAb directed against a pan-HLA class II DR epitope. The T cells were then added to target cells, and IFN-γ release measured following an overnight incubation.

### CIITA transduction of tumour lines.

In order to induce stable expression of cell surface MHC class II molecules, the tumour lines 1869 col, SW480, and Colo205 were transduced with a recombinant retrovirus that was generated by cloning the gene that encoded the human class II transactivator (CIITA) into the retroviral expression vector pCLRCX (17). The transduced 1869 tumour cells were then sorted using a FACSVantage^{™} cell sorter (Becton Dickinson) to obtain cells that homogeneously expressed relatively high levels of cell surface HLA class II expression.

### Isolation of MHC class II DRβ1 molecules.

The DRβ1*0402 gene was isolated by carrying out an RT-PCR with RNA derived from the tumour line 1869 col, and the DRβ1*1301 gene was obtained by carrying out an RT-PCR with RNA derived from an autologous T cell line. Primers that were used to amplify HLA-DR were: 5'-TCCAGCATGGTGTGTCTGA-3' (SEQ ID NO 13) and 5'-CCTTGAATGTGGTCATCT-3' (SEQ ID NO 14). Two additional primers were designed to specifically amplify the HLA-DR13 gene product: 5'CGTTTCTTGGAGTACTCTACGTC-3' (SEQ ID NO 15) and 5'-CCACCGCGGCCCGCTCGTCT-3' (SEQ ID NO 16). The isolated products were cloned in the plasmid vector pCR-Blunt (Invitrogen, Carlsbard, CA) and sequenced using an ABI Prism 310 Genetic analyser (Perkin-Elmer, Shelton, CT). The genes were then cloned in the eukaryotic expression vectors pCDNA3.1 (Invitrogen) and the retroviral expression vector CLRCX4, discussed above.

Constructs encoding either of the HLA-DRβ1 genes were co-transfected along with a construct encoding the HLA-DRα gene into 293 cells. Stable transfectants were stained with the FITC labelled anti-HLA-DR mAb L243, and cells that were strongly positive for the expression of the cell surface HLA-DR molecules were isolated using a FACSVantage^{™} cell sorter (Becton Dickinson). To induce the expression of molecules involved with HLA class II antigen processing, such as the class II invariant chain, DMA, and DMB genes, the 293 cells that had been transfected with the HLA-DR constructs were then transduced with recombinant retroviral supernatants generated using the CLRC-CIITA construct, as previously described (17).

### cDNA Library Construction and screening.

Total RNA was extracted from 1869 col tumour line using Triazol (GIBCO, BRL) and poly (A) RNA was then isolated using poly (A) Tract (Promega, Madison, WI). The poly (A) RNA was then converted to cDNA using the SuperScript cDNA Synthesis kit (InVitrogen) and cloned in the episomal mammalian expression vector pEAK8 (Edge BioSystems, Gaithersburg, MD). The pEAK8 vector had been modified by cloning a fragment encoding amino acids one to 80 of the human invariant chain (Ii) downstream of the EF1-α promoter in order to express the cDNA inserts as fusion constructs and target the gene products to the HLA class II antigen presentation pathway. The recombinant cDNA was then electroporated into DH10B electrocompetent cells (InVitrogen), and plasmid pools containing approximately 50 cDNA recombinants prepared as previously described (18). The 293 cell lines that were transfected with HLA-DRβ1*0402 (293-DR0402) or HLA-DRβ1*1301 (293-DR13) were transiently transfected with DNA prepared from the cDNA pools (200 ng) using Lipofectamine 2000 (InVitrogen) according to the manufacturer's directions.

In order to conserve C111 T cells, screening assays were initially carried out by transfecting a mixture of 5x10⁴ 293-DR*0402 and 5x10⁴293-DR*1301 cells with cDNA library pools in 96 well flat bottom plates. The following day the cells were washed and 1x10⁵ cells T cells in AIM-V medium plus 2 % HS were added each well. After 18 hrs of incubation at 37°C and 5 % CO₂, 100 µl of supernatant was collected and the IFN-γ release was evaluated by ELISA. For subsequent assays, cDNA pools and clones were transfected into 293 cells that expressed only a single HLA DR allele, and these cells were tested for their ability to stimulate C111 T cells.

### 5' Rapid amplification of cDNA ends (RACE).

Total RNA was extracted from the 1869 col tumour cell line and a 5' RACE was performed using the Smart RACE cDNA amplification kit according the manufacturer's instructions (Clontech, Franklin Lakes, NJ). The RT-PCR products were cloned into the pCDNA 3.1 Topo cloning vector (Invitrogen) and recombinant DNA was prepared for sequence analysis. In addition, amplification of the full length COA-1 gene products was carried out using the Advantage 2 PCR kit (Clontech). The amplification was carried out by incubation at 95°C for 1 minute, followed by 35 amplification cycles consisting of a 30 second incubation at 95°C, a 30 second annealing step at 62°C, and a 2 minute extension step at 68°C.

### Identification of T cell epitopes.

Peptides of 20 or 21 amino acids in length that overlapped by 15 amino acids that were encoded by the long open reading frame of the original cDNA clone that was isolated were synthesised by solid-phase method using a peptide synthesiser (AMS 422; Gilson Co., Inc. Middleton, WI). The purity of the peptides was verified by mass spectrometry (Tuft's Core Facility, Boston, MA). Allogeneic B cells (1x10⁵ cells/well) that expressed either the DRβ1*0402 or the DRβ1*1301 molecules were incubated with 50 µg/ml in 100 µl/well of ISCOVE'S medium plus 10 % HS in flat bottom-96-well plates. After three hours, 1-5x10⁴ T cells were added to the wells in 150 µl/well of medium and incubated for 18 hours at 37°C and 5% CO2, followed by measurement of INF-γ release by ELISA..

### Results

### Generation and characterization of colon cancer lines.

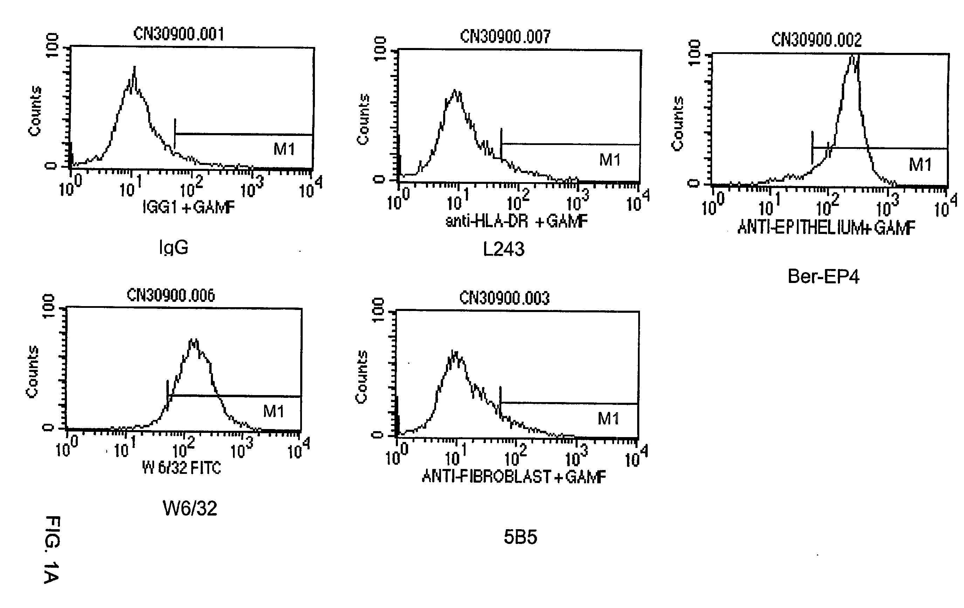

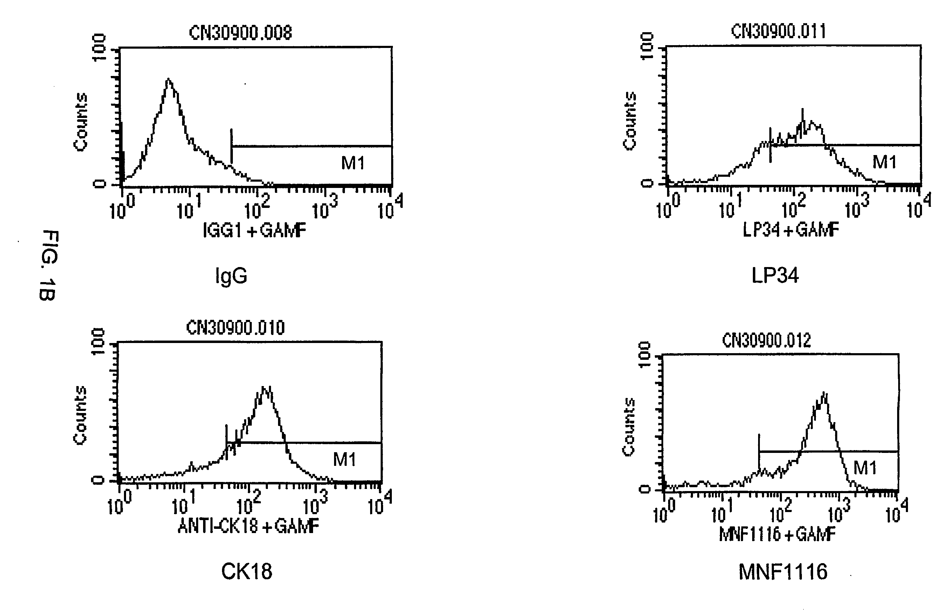

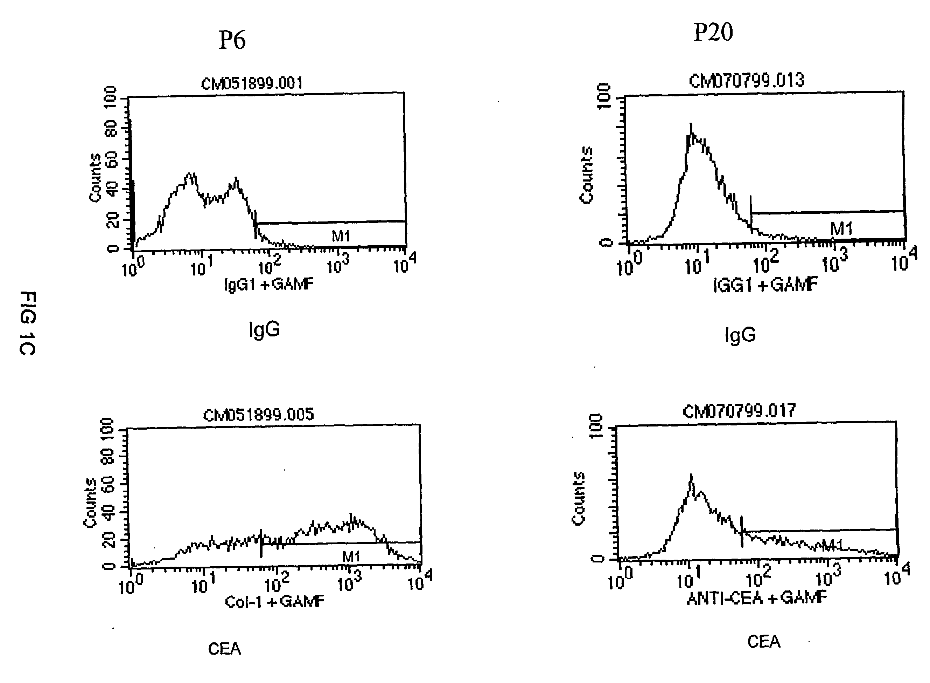

Cultured colon cancer lines were initially established from liver metastasis specimens obtained from five colorectal cancer patients. Analysis of one of the most rapidly proliferating cell lines that was obtained, 1869 col, demonstrated that these cells expressed a common epithelial marker, expressed cytokeratins associated with epithelial cells (Fig.1), and maintained a morphology in tissue culture that was typical of epithelial cells (data not shown).

In contrast, the cell lines did not stain with an antibody directed against the β subunit of prolyl-4-hydroxylase, a cell surface marker expressed in fibroblasts. Taken together, these results indicated that these cells were of epithelial origin and represented colon cancer cell lines and did not contain significant numbers of normal cells. The 1869 col cell line expressed uniform levels of MHC class I molecules and low or undetectable levels of cell surface MHC class II molecules were found on the same cells (Fig. 1), but treatment of the 1869 col cells with IFN-γ resulted in strong up-regulation of HLA class II expression (data not shown).

The carcinoembryonic antigen represents a marker that is expressed at high levels *in vivo* on colon tumour cells as well as on many colon tumour cell lines, but is not expressed by fibroblasts or hepatic cells. Analysis of 1869 col cells indicated that they expressed CEA (Fig. 1), and the additional colon tumour cell lines that were generated appeared to express similar levels of this gene product (data not shown). An early passage of the 1869 col cell line demonstrated high level expression of CEA, and lower but still significant levels of CEA expression were observed at later passages of 1869 col cells (Fig. 1). These observations are consistent with previous studies in which heterogeneous expression of CEA was observed on a variety of colon tumour cell lines (19).

### Isolation and characterization of colon cancer reactive T lymphocytes.

In the initial attempts to derive colon tumour reactive T cells, tumour infiltrating lymphocytes (TIL) from patient 1869 were cultured in high dose IL-2. In addition, autologous tumour cells, that had been treated with IFN-γ to up-regulate HLA class II gene expression, were used to carry out in vitro mixed lymphocyte tumour cultures (MLTC) with PBMC from patient 1869. Three CD4⁺ tumour reactive T cell clones, C4, C49 and C111, were initially selected for further analysis on the basis of their high degree of reactivity with the autologous tumour cell line.

The three clones derived from PBMC released IFN-γ in response to autologous tumour cells that had been treated with IFN-γ, and these clones released significantly higher levels of IFN-γ in response to 1869 tumour cells that had been treated with the CIITA and sorted for cells that constitutively expressed high levels of cell surface HLA class II molecules (Table 2).

Relatively low levels of IFN-γ were released following stimulation with the autologous 1869 EBV B cell line from the three T cell clones. All of the T cell clones released IFN-γ and GM-CSF but not IL-4 following stimulation with HLA class II positive tumour cells (data not shown), indicating that they represent cells of the Th1 cell phenotype.

In order to test whether the clones isolated from the PBMC recognised tumour cells in an MHC-restricted manner, cytokine release assays were carried out in the presence of anti-HLA class I and class II specific antibodies using stimulator cells bearing a variety of MHC haplotypes (Table 1). The results indicated that the C4, C49 and C111 T cell clones recognised the autologous tumour cells in the context of the HLA DR class II restriction element (Table 2). The C49 and C111 T cell clones also recognised the CIITA transduced allogeneic MHC class II⁺ colon cancer lines SW480 and Colo 205 that shared expression of HLA-DRβ1*1301 with the autologous tumour, and this recognition was blocked by pre-incubation of the tumour cell lines with the anti-HLA-DR mAb.

Generally the responses were inhibited by between 50 and 90% by pre-incubation with the anti-HLA DR antibody, whereas less than 20% inhibition was observed with the anti-HLA class I antibody. The response of the C4 line to the SW480 CIITA treated tumour cell lines, as well as the response of C111 to theColo205 CIITA, were only partially inhibited by anti-HLA DR antibody, which might reflect the fact that these T cells can recognise additional ligands other than the classical TCR. The C4, C49 and C111 clones recognised autologous EBV B cells as well as an allogeneic EBV B cell line that shared expression of HLA DRβ1*1301 with autologous cells. Normal B cells that were generated by stimulating autologous PBMC with CD40 ligand plus IL-4, as well as an allogeneic fibroblast cell line that shared expression of HLA DRβ1*1301 with the 1869 col tumour and that was treated with IFN-γ to up-regulate HLA class II gene expression, stimulated little or no cytokine release from these T cells (Table 3).

Two CD4+ T cell clones from TIL 1869 that responded in preliminary assays to autologous HLA class II positive tumour cells were also tested for their ability to recognise autologous as well as allogeneic colon tumour cell lines. Clones C4, C49 and C111, as well as two clones derived from 1869 TIL, C5 and C15, responded to the allogeneic colon tumour cell line 1847 col that shared expression of the HLA-DRβ1*1301 gene product with the autologous tumour. In contrast, the allogeneic 1872 col cell line that did not share expression of any HLA DR gene products with the 1869 col tumour failed to stimulate significant cytokine release from the T cell clones.

### Identification of the antigen recognised by C111 T cells.

Further studies aimed at identifying tumour antigens expressed on 1869 col cells focused on C111 T cells, which was the only T cell clone that expanded sufficiently to allow the cDNA library to be screened. The results of studies carried out with additional tumour histologies indicated that C111 T cells did not recognise two allogeneic renal cell lines, as well as a prostate tumour cell line that shared expression of HLA-DRβ1*1301 with the 1869 col cell line (data not shown). A single allogeneic melanoma cell line that expressed HLA-DRβ1*0402 was identified, 1681 mel. Cell surface HLA class II expression was up-regulated following treatment of the 1681 mel cell line with IFN-γ, and the treated cells were recognised by C111 T cells, indicating that certain tumour types shared expression of the antigen recognised by these T cells (Table 3).

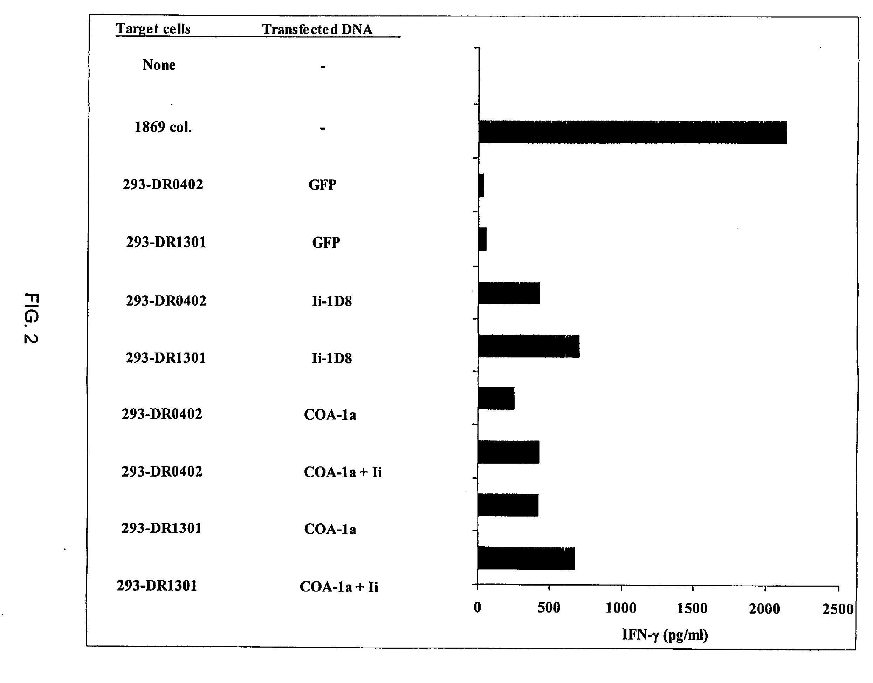

Stable transfectants of the 293 cell line that expressed either the autologous MHC class II DRβ1*0402 or 1301 gene products molecules were then mixed in equal numbers and transiently transfected with DNA pools generated from the autologous tumour cell cDNA library. The positive pool that was initially identified following the screening of approximately 3x10⁴ clones, 4G3, appeared to sensitise either 293-DRβ1*0402 or 1301 target cells for recognition by C111 T cells, and a single cDNA clone that could sensitise target cells for recognition by C111 T cells, 1D8, was identified (Fig. 2).

An assay carried out by transfection of the 293-DRβ1*0402⁺ or 1301⁺ cell lines individually with the 1D8 cDNA indicated that either of these HLA class II restriction elements could present the T cell epitope to C111 T cells. In contrast, 293 cell lines that expressed the HLA-DRβ1*0101, 0401, 0701 or 1601 class II alleles failed to stimulate these T cells following transfection of the 1D8 cDNA clone (data not shown), indicating that presentation of this epitope to C111 T cells may be limited to the two autologous HLA-DR alleles expressed by 1869 col cells. Further screening of the cDNA library resulted in the isolation of a second cDNA clone that was nearly identical to the 1D8 clone. The isolation of a second clone with a nearly identical sequence supports the finding that this represents the natural transcript encoding the antigen recognised by C111 T cells

### Characterization of colorectal tumour associated antigen COA-1.

The 1D8 insert contained a 44 bp polyA tail at the 3' end, but appeared to represent a partial cDNA clone as it was only 291 bp in length. The 5' end of the gene product that was expressed in the 1869 col cell line was then isolated by carrying out a rapid amplification of cDNA ends (RACE) reaction using nested internal primers complementary to the sequence of the 1D8 clone. Sequencing of products that were cloned from this reaction indicated that a 1412 bp product represented the predominant transcript of the gene in the 1869 col cell line that encoded the antigen recognised by C111 T cells, which was designated colorectal antigen-1 (COA-1) (Fig 3).

Comparison of the COA-1 sequence with the genomic DNA sequence database indicated that this product was derived from 13 exons, but at least two additional alternatively spliced products of this gene were isolated from the RACE reaction. An alignment of the COA-1 transcript with the human EST database indicated that this was identical or nearly identical to several sequences obtained from normal human brain, placenta, ovary, and testis, as well as sequences obtained from a variety of adenocarcinomas.

The 5' end of the transcript cloned from the RACE reaction corresponded to the 5' end of several EST sequences found in the database, and the 3' end of the original cDNA clone corresponded to the 3' end of the EST transcripts derived from several cell lines, indicating that these may represent the authentic 5' and 3' ends of the predominant COA-1 colon tumour cell transcript. The COA-1 sequence was also nearly identical to that of a transcript encoding the human homologue of the rat Socius protein, a molecule that was recently cloned on the basis of its ability to bind to a member of the Rnd family of GTPases (20).

Forward and reverse primers located at or near the 5' and 3' ends of the putative COA-1 gene product were then used to carry out an RT-PCR from 1869 RNA, as the RACE products that had been cloned only comprised a portion of the normal transcript. When RT-PCR was carried out with several primers that were proximal to the putative 5' end of the transcript in combination with primers that were complementary to the highly repetitive G/C rich sequence near to the 3' end of the COA-1 transcript, a variety of non-specific transcripts were generated (data not shown). A product that was designated COA-1a was, however, successfully amplified from 1869 col RNA using two primers that encompassed the region between nucleotides 290 and 1318 of the putative full length COA-1 transcript.

Transfectants that co-expressed the COA-1a gene along with either HLA-DRβ1*0402 or 1301, appeared to stimulate comparable levels of cytokine release from C111 T cells to those transfected with the truncated 1D8 cDNA clone, showing that the full length gene can be processed relatively efficiently (Fig. 2). Co-transfection of the COA-1a gene with a construct encoding the full length human invariant chain (Ii) had little or no effect on the recognition of target cells transfected with the COA-1a product by C111 T cells. Thus, either the levels of Ii expression in 293 cells that were also transfected with a construct encoding the CIITA gene product was adequate for recognition of this epitope, or Ii expression does not have a significant impact on the processing of the COA-1 epitope.

In addition, the COA-1a product was not fused with amino acids one to 80 of the human Ii molecule, which had previously been shown to enhance the recognition of some HLA class II antigens (21).

The observation that the fusion of the cDNA clone with the invariant chain did not enhance recognition by the CD4+ T cells shows that the COA-1 antigen may naturally target the endogenous HLA class II processing pathway in colon tumour cells.

The expression pattern of the COA-1 gene was then examined in several colorectal, melanoma, and EBV-B cell lines, as well as in several normal cell lines which included CD40L stimulated B cell and fibroblast cell lines. The results of Northern blot analysis indicated that this gene was expressed at relatively low levels in colon and melanoma tumour cell lines, EBV B cells, normal B cells and fibroblasts, and quantitative TaqMan RT-PCR indicated that the levels of expression did not differ significantly between these cells (data not shown).

The observation that the level of expression of the COA-1 gene did not differ significantly between cell lines that were or were not recognised by C111 T cells, showed that these cells express similar but non-identical products. Therefore, transcripts of the COA-1 gene that were expressed in the autologous and allogeneic CD40L stimulated B cells, as well as allogeneic fibroblast cell lines, were isolated using RT-PCR and sequenced.

The results of sequencing carried out with the bulk RT-PCR products showed that CD40L stimulated B cells and fibroblast cell lines predominantly expressed products that appeared to be identical to the COA-1 transcript derived from 1869 col cells with the exception of a single substitution of a T for a C residue at nucleotide position 1280, resulting in a change at amino acid 399.

The COA-1 transcripts that were expressed in CD40L B cells were isolated by carrying out RT-PCR and cloning the resultant products. Ten out of ten clones from the CD40L B cells that were sequenced contained a T at position 1280 but were otherwise identical to the 1869 col COA-1 transcript.

Amplification of the COA-1 gene product from allogeneic colorectal tumour lines SW1463, SW480 and 1847 col, as well as the 1681 mel line, showed that these cells predominantly expressed products containing a C residue at position 1280, as determined by sequencing the bulk, un-cloned RT-PCR products that were amplified from these cells (data not shown). Two peaks of comparable heights that corresponded to C and T residues at position 1280 of the COA-1 transcript were derived by sequencing the un-cloned RT-PCR product from autologous EBV B cells, indicating that these products may be expressed at similar levels in these cells. The results obtained using RNA from autologous CD40L stimulated B cells, EBV B cells, and the colon tumour cell lines were confirmed by repeated analysis carried out on products obtained from four independent RT-PCR reactions, showing that the residue found at nucleotide 1280 of the COA-1 transcripts did not represent a PCR mutation (data not shown).

To evaluate the significance of the single base pair change at position 1280 in the COA-1a sequence, the RT-PCR products obtained from autologous CD40L stimulated B cells were cloned in a eukaryotic expression vector. A plasmid containing the COA-1a transcript that was amplified from the normal B cells was then compared with products cloned from 1869 col cells for its ability to sensitise 293-DR*0402 or 293-DR*1301 cells for recognition by C111 T cells. Target cells expressing either of the autologous HLA-DR genes that were transfected with the COA-1a or 1D8 gene products, but not the product that was isolated from CD40L activated B cells, stimulated cytokine release from C111 T cells (Fig. 4). These results showed that there was a correlation between the recognition of normal B cells and tumour cells and the ability of the COA-1 gene products that were expressed by these cells to sensitise targets for recognition by C111 T cells.

### Identification of the epitope recognised by the CD4⁺ clone C111.

The results of transfection studies carried out using truncated COA-1 gene products showed that the C111 T cell epitope was encoded by a region located between nucleotides 1121 and 1288 of the COA-1 transcript. The longest open reading frame in the COA-1 transcript, which overlapped with the Socius gene product (20), was utilised as the basis for the synthesis of peptides that were used to identify the T cell epitope recognised by C111 T cells.

Peptides that were 20 or 21 amino acids in length and that overlapped by either 14 or 16 amino acids, were than synthesised and tested for their ability to sensitise target cells for recognition by C111 T cells. Since autologous normal B cells could not be efficiently expanded, allogeneic normal B cells expressing either DRβ1*0402 or DRβ1*1301 were used to carry out these assays.

The 1681 and 1847 CD40L stimulated normal B cell lines shared expression of HLA-DRβ1*0402 and HLA-DRβ1*1301 molecules, respectively, with the autologous tumour cell line. These cells were incubated with the panel of peptides and then tested for their ability to stimulate cytokine release from C111 T cells. The results showed that 1681 and 1847 CD40L B cells that were pulsed with either of the two overlapping peptides FSTFPPTLYQDDTLTLQAAG (SEQ ID NO 17) and TLYQDDTLTLQAAGLVPKAA (SEQ ID NO 18) stimulated significant cytokine release from C111 T cells.

These T cells thus recognise the peptide TLYQDDTLTLQAAG (SEQ ID NO 6), which represents the overlapping region in these peptides. The L at position two, the T at the position 7 and L at position 10 in this sequence conform to an HLA binding motif that has been identified for the HLA-DRβ1 *0402 class II allele (22). However, it was not possible to identify the potential anchor residues in this sequence that were involved in binding to the HLA-DRβ1*1301 allele. Nevertheless, these observations show that C111 T cells recognise a single peptide epitope in the context of either the HLA-DRβ1 *0402 or 1301 class II gene products.

### REFERENCES

1. DeCosse, J. J., Tsioulias, G.J., and Jacobson, J.S. Colorectal cancer: detection, treatment, and rehabilitation. CA Cancer J. Clin., 44: 27-42, 1994.
2. Harrington, D. P. The tea leaves of small trials. J Clin Oncol, 17: 1336-1338., 1999.
3. Rosenberg, S. A., Packard, B. S., Aebersold, P. M., Solomon, D., Topalian, S. L., Toy, S. T., Simon, P., Lotze, M. T., Yang, J. C., Seipp, C. A., Simpson, C., Carter, C., Bock, S., Schwartzentruber, D., Wei, J. P., and White, D. E. Use of tumour infiltrating lymphocytes and interleukin-2 in the immunotherapy of patients with metastatic melanoma. Preliminary report. N Engl J Med, 319: 1676-1680, 1988.
4. Mukherji, B. and Chakraborty, N. G. Immunobiology and immunotherapy of melanoma. Curr Opin Oncol, 7: 175-184., 1995.
5. Riethmuller, G., Holz, E., Schlimok, G., Schmiegel, W., Raab, R., Hoffken, K., Gruber, R., Funke, I., Pichhnaier, H., Hirche, H., Buggisch, P., Witte, J., and Pichlmayr, R. Monoclonal antibody therapy for resected Dukes' C colorectal cancer: seven-year outcome of a multicenter randomized trial. J Clin Oncol, 16: 1788-1794., 1998.
6. Vermorken, J. B., Claessen, A. M., van Tinteren, H., Gall, H. E., Ezinga, R., Meijer, S., Scheper, R. J., Meijer, C. J., Bloemena, E., Ransom, J. H., Hanna, M. G., Jr., and Pinedo, H. M. Active specific immunotherapy for stage II and stage III human colon cancer: a randomised trial. Lancet, 353: 345-350., 1999.
7. Naito, Y., Saito, K., Shiiba, K., Ohuchi, A., Saigenji, K., Nagura, H., and Ohtani, H. CD8+ T cells infiltrated within cancer cell nests as a prognostic factor in human colorectal cancer. Cancer Res, 58: 3491-3494., 1998.
8. Browning, M., Petronzelli, F., Bicknell, D., Krausa, P., Rowan, A., Tonks, S., Murray, N., Bodmer, J., and Bodmer, W. Mechanisms of loss of HLA class I expression on colorectal tumour cells. Tissue Antigens, 47: 364-371., 1996.
9. Garrido, F., Cabrera, T., Concha, A., Glew, S., Ruiz-Cabello, F., and Stern, P. L. Natural history of HLA expression during tumour development. Immunol Today, 14: 491-499., 1993.
10. Coulie, P. G., Ikeda, H., Baurain, J. F., and Chiari, R. Antitumor immunity at work in a melanoma patient. Adv Cancer Res, 76: 213-242, 1999.
11. Renkvist, N., Castelli, C., Robbins, P. F., and Parmiani, G. A listing of human tumour antigens recognized by T cells. Cancer Immunol Immunother, 50: 3-15., 2001.
12. Tsang, K. Y., Zhu, M., Nieroda, C. A., Correale, P., Zaremba, S., Hamilton, J. M., Cole, D., Lam, C., and Schlom, J. Phenotypic stability of a cytotoxic T-cell line directed against an immunodominant epitope of human carcinoembryonic antigen. Clin Cancer Res, 3: 2439-2449., 1997.
13. Akagi, J., Nakagawa, K., Egami, H., and Ogawa, M. Induction of HLA-unrestricted and HLA-class-II-restricted cytotoxic T lymphocytes against MUC-1 from patients with colorectal carcinomas using recombinant MUC-1 vaccinia virus. Cancer Immunol Inmunother, 47: 21-31., 1998.
14. Brossart, P., Stuhler, G., Flad, T., Stevanovic, S., Rammensee, H. G., Kanz, L., and Brugger, W. Her-2/neu-derived peptides are associated-associated antigens expressed by human renal cell and colon carcinoma lines and are recognized by in vitro induced specific cytotoxic T lymphocytes. Cancer Res, 58: 732-736., 1998.
15. Ito, M., Shichijo, S., Tsuda, N., Ochi, M., Harashima, N., Saito, N., and Itoh, K. Molecular basis of T cell-mediated recognition of pancreatic cancer cells. Cancer Res, 61: 2038-2046., 2001.
16. Lapointe, R., Lemieux, R., Olivier, M., and Darveau, A. Tyrosine kinase and cAMP-dependent protein kinase activities in CD40- activated human B lymphocytes. Eur J Immunol, 26: 2376-2382., 1996.
17. Robbins, P. F., El-Gamil, M., Li, Y. F., Zeng, G., Dudley, M., and Rosenberg, S. A. Multiple HLA Class II-Restricted Melanocyte Differentiation Antigens Are Recognized by Infiltrating-Infiltrating Lymphocytes from a Patient with Melanoma. J Immunol, 169: 6036-6047, 2002.
18. Robbins, P. F., El-Gamil, M., Li, Y. F., Kawakami, Y., Loftus, D., Appella, E., and Rosenberg, S. A. A mutated β-catenin gene encodes a melanoma-specific antigen recognized by tumour infiltrating lymphocytes. J.Exp.Med., 183: 1185-1192, 1996.
19. Lopez-Conejo, T., Olmo, N., Turnay, J., Navarro, J., and Lizarbe, A. Characterization of tumorigenic sub-lines from a poorly tumorigenic human colon-adenocarcinoma cell line. Int J Cancer, 67: 668-675, 1996.
20. Katoh, H., Harada, A., Mori, K., and Negishi, M. Socius is a novel Rnd GTPase-interacting protein involved in disassembly of actin stress fibers. Mol Cell Biol, 22: 2952-2964, 2002.
21. Wang, R. F., Wang, X., Atwood, A. C., Topalian, S. L., and Rosenberg, S. A. Cloning genes encoding MHC class II-restricted antigens: mutated CDC27 as a tumour antigen. Science, 284: 1351-1354, 1999.
22. Rammensee, H., Bachmann, J., Emmerich, N. P., Bachor, O. A., and Stevanovic, S. SYFPEITHI: database for MHC ligands and peptide motifs. Immunogenetics, 50: 213-219, 1999.
23. Topalian, S. L., Gonzales, M. I., Parkhurst, M., Li, Y. F., Southwood, S., Sette, A., Rosenberg, S. A., and Robbins, P. F. Melanoma-specific CD4+ T cells recognize nonmutated HLA-DR-restricted tyrosinase epitopes. J.Exp.Med., 183: 1965-1971, 1996.
24. Morel, S., Levy, F., Burlet-Schiltz, O., Brasseur, F., Probst-Kepper, M., Peitrequin, A. L., Monsarrat, B., Van Velthoven, R., Cerottini, J. C., Boon, T., Gairin, J. E., and Van den Eynde, B. J. Processing of some antigens by the standard proteasome but not by the immunoproteasome results in poor presentation by dendritic cells. Immunity, 12: 107-117,2000.
25. Doherty, D. G., Penzotti, J. E., Koelle, D. M., Kwok, W. W., Lybrand, T. P., Masewicz, S., and Nepom, G. T. Structural basis of specificity and degeneracy of T cell recognition: pluriallelic restriction of T cell responses to a peptide antigen involves both specific and promiscuous interactions between the T cell receptor, peptide, and HLA-DR. J Immunol, 161: 3527-3535., 1998.
26. Di Giorgio, A., Botti, c., Tocchi, A., Mingazzini, P., and Flammia, M. The influence of tumour lymphocyte infiltration on long term survival of surgically treated colrectal cancer patients. Int. Surg., 77: 256-260, 1992.
27. Kinihiro, M., Tanaka, S., Haruma, K., Yoshihara, M., Sumii, K., Kajiyama, G., Shimamoto, F. Combined expression of HLA-DR antigen and proliferating cell nuclear antigen correlate with colorectal cancer prognosis. Oncology, 55: 326-333, 1998.
28. Ransom, J. H., Pelle, B., and Hanna, Jr., M.G. Expression of class II major histocompatibility complex molecules correlates with human colon tumour vaccine efficacy. Can. Res., 52: 3460-3466, 1992.
29. Yang, D., Nakao, M., Shichijo, S., Sasatomi, T., Takasu, H., Matsumoto, H., Mori, K., Hayashi, A., Yamana, H., Shirouzu, K., and Itoh, K. Identification of a gene coding for a protein possessing shared tumour epitopes capable of inducing HLA-A24-restricted cytotoxic T lymphocytes in cancer patients. Cancer Res, 59: 4056-4063., 1999.
30. Bremers, A. H. A., Andreola, S., Leo, E., Gallino, F., Rini, F., Lombardo, C., Belli, F., Kuppen, P.J.K., Parmiani, G., and Castelli, C. T cell responses in colorectal cancer patients: evidence for class-II HLA restricted recognition of shared associated-associated antigens. Int. J. Can., 88: 956-961, 2000.
31. Saeterdal, I., Bjorheim, J., Lislerud, K., Gjertsen, M. K., Bukholm, I. K., Olsen, O. C., Nesland, J. M., Eriksen, J. A., Moller, M., Lindblom, A., and Gaudernack, G. Frameshift-mutation-derived peptides as specific-specific antigens in inherited and spontaneous colorectal cancer. Proc Natl Acad Sci U S A, 98: 13255-13260., 2001.

**Table 1 MHC Haplotype of cell lines**

| | **A** | **B** | **C** | **DRβ1** | **DRβ3-5** | **DQ** |
|---|---|---|---|---|---|---|
| **1869** | 3,24 | 35,38 | 0401,1203 | 0402,1301 | 3*01,4*01 | 03,06 |
| **1870** | 24 | 35 | 04 | 1202 | 3*03 | 03 |
| **1872** | 02,03 | 07,4402 | 0501, 0702 | 0401,1501 | 4*01, 5*01 | 03,06 |
| **1681** | 01,0201 | 08,44 | N.D. | 0301,0402 | 3*0101,4*01 | 0301,0402 |
| **1847** | 02 | 18,44 | 05, 0701 | 0401,1301 | 3*01, 4*01 | 03,06 |
| **1519** | 24,32 | 1401,4402 | 05,08 | 0701,1301 | 3*01,4*01 | 02,06 |

**Table 2 Specific recognition of colon cancer lines by CD4⁺ clones from patient 1869.**

| Target cells | Antibody^{a} | HLA-DRβ1 | T cell^{b} | | |
|---|---|---|---|---|---|
| | | | C4 | C49 | C111 |
| None | None | | < 8^{c} | < 8 | < 8 |
| 1869 col + 1FN-γ^{d} | None | *0402, *1301 | 234 | 1213 | 536 |
| | W6/32 | | 212 | 1100 | 442 |
| | L243 | | 107 | 97 | 17 |
| 1869 col CIITA | None | *0402, *1301 | 536 | 5178 | 5005 |
| | W6/32 | | 527 | 4987 | 4249 |
| | L243 | | 47 | 254 | 305 |
| 1870 col + IFN-γ | None | *1202 | < 8 | < 8 | < 8 |
| | W6/32 | | < 8 | <8 | <8 |
| | L243 | | < 8 | < 7.8 | < 8 |
| 1872 col + IFN-γ | None | *0401, *1501 | < 8 | < 8 | < 8 |
| | W6/32 | | <8 | <8 | <8 |
| | L243 | | <8 | <8 | <8 |
| SW 480 CIITA | None | *0103, *1301 | 879 | 968 | 963 |
| | W6/32 | | 780 | 902 | 996 |
| | L243 | | 571 | 129 | 127 |
| Colo 205 CITTA | None | *0401, *1301 | 68 | 942 | 686 |
| | W6/32 | | 76 | 951 | 669 |
| | L243 | | 78 | 170 | 489 |
| 1869 EBV-B | | *0402, *1301 | 52 | 126 | 322 |

| | | | | | |
|---|---|---|---|---|---|
| a.Target cells were pre-incubated for 1 hour with either the anti-MHC class I mAb W6/32 or the anti-HLA DR mAb L243 before addition to T cells. b. 2x10⁴ T cells were incubated with 5x10⁴ target cells in flat bottom 96-well plate in 250 µl of AIMV 2% HS. After 18 hrs. the supernatants IFN-γ secretion was evaluated by ELISA. c. pg/ml of IFN-γ. d. Where indicate, target cells were pre-incubated for 48 hrs with 500 IU of IFN-γ. | | | | | |

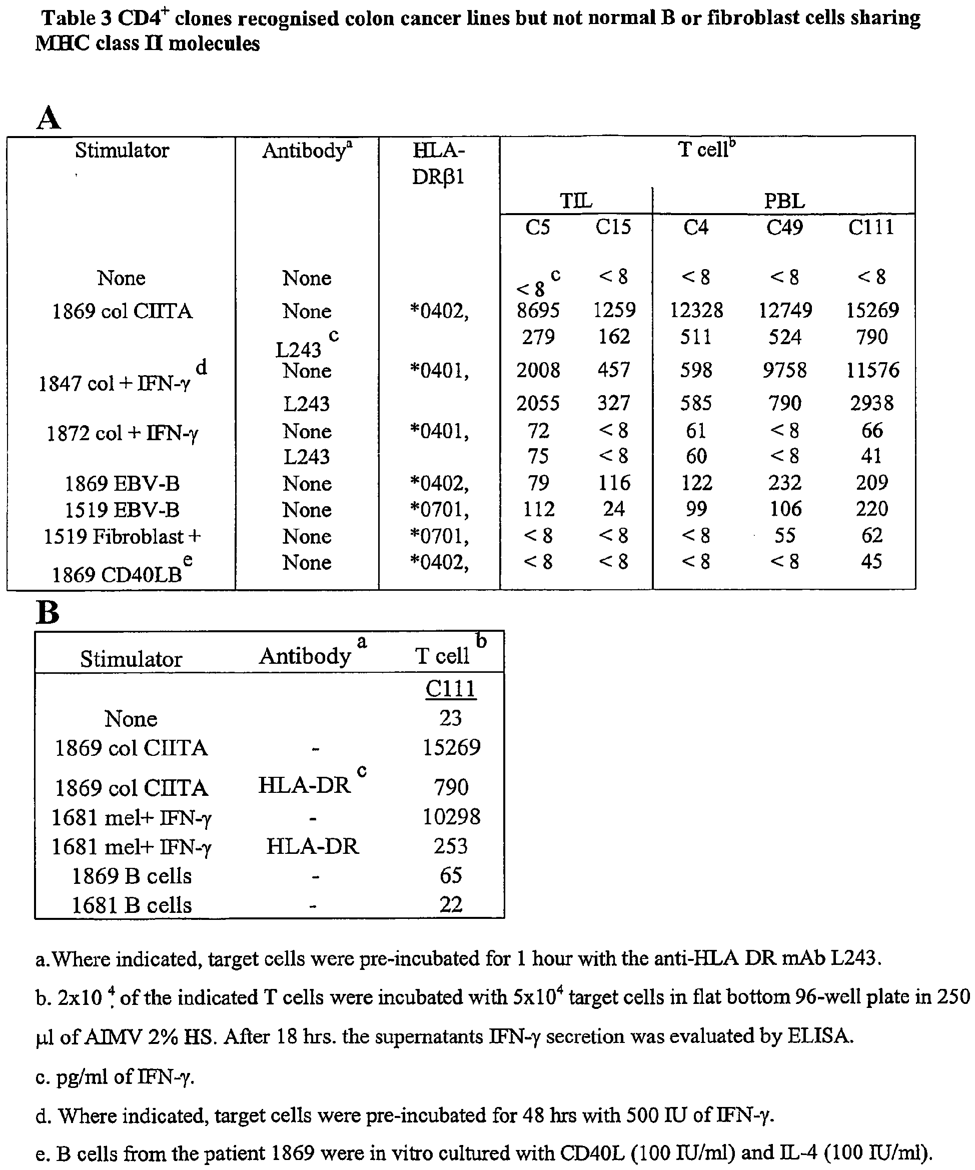

**Table 4 Identification of the COA-1-derived epitopes recognised by the CD4⁺ clone C111.**

| **Stimulator** | **HLA-DRβ1** | **No peptide** | | | | | |
|---|---|---|---|---|---|---|---|
| None | | <8^{a} | | | | | |
| 1869 col | 0402, | 2186 | | | | | |
| | 1301 | | | | | | |
| 1681 | 0301, | <8 | | | | | |
| CD40LB | 0402 | | | | | | |
| 1847 | 0401, | <8 | | | | | |
| CD40LB | 1301 | | | | | | |

| | | **Peptide ^{b}** | | | | | |
|---|---|---|---|---|---|---|---|
| | | | **Peptide Conc. (µg/ml)** | | | | |
| | | | **100** | **50** | **25** | **12.5** | **6.25** |
| 1681 CD40LB | 0301, 0402 | FSTFPPTLYQDDTLTLQAAG | 105 | 236 | 69 | <7.8 | <7.8 |
| 1681 CD40LB | | TLYQDDTLTLQAAGLVPKAA | 51 | 159 | <7.8 | <7.8 | <7.8 |
| 1681 CD40LB | | DDTLTLQAAGLVPKAALLLRA | 11 | 16 | <7.8 | <7.8 | <7.8 |
| 1681 CD40LB | | LQAAGLVPKAALLLRARRAP | 21 | 12 | <7.8 | <7.8 | <7.8 |
| | | | | | | | |
| 1847 CD40LB | 0401, 1301 | ASAFEIFSTFPPTLYQDDTL | <7.8 | <7.8 | <7.8 | <7.8 | <7.8 |
| 1847 CD40LB | | FSTFPPTLYQDDTLTLQAAG | 226 | 397 | 296 | 79 | <7.8 |
| 1847 CD40LB | | TLYQDDTLTLQAAGLVPKAA | 79 | 326 | <7.8 | <7.8 | <7.8 |
| 1847 CD40LB | | DDTLTLQAAGLVPKAALLLRA | 22 | 33 | <7.8 | <7.8 | <7.8 |
| 1847 CD40LB | | LQAAGLVPKAALLLRARRAP | 52 | 32 | <7.8 | <7.8 | <7.8 |

| | | | | | | | |
|---|---|---|---|---|---|---|---|
| a. The CD4⁺ T cell clone C111 was the added at 2x10⁴ cells/well at the final volume of 250 µl/well of ISCOVE's plus 10 % HS and after 18 hrs. of incubation the supernatants were collected and the IFN-γ release was evaluated by ELISA. b. Peptides of 20 or 21 amino acids overlapping by 15 amino acids were synthesised using the putative COA-1 protein, in the 1D8 region (1012-1318 bp). 4x10⁵/ml of B cells sharing one of the DRβ1 molecules (*0402 or *1301) with the autologous tumour 1869, were incubated for three hrs. at 37°C and 5% CO₂ in the presence or not (-) of the peptides at the final volume of 100 µl/well in ISCOVE's plus 10% HS. | | | | | | | |

## Claims

1. A peptide, comprising all or an immunogenic part of the amino acid sequence designated SEQ ID NO 6, for use in a method of vaccine treatment of colorectal cancer or melanoma by stimulating an anti-colorectal cancer or an anti-melanoma immune response against COA-1 (SEQ ID NO 2), wherein the immunogenic part of the sequence is processed and expressed by antigen presenting cells in association with sympathetic MHC class II molecules.

2. A peptide according to claim 1, wherein the immunogenic part of the sequence comprises 8 or more contiguous amino acid residues of SEQ ID NO 6.

3. A peptide according to claim 2, wherein the immunogenic part of the sequence comprises 10 or more contiguous amino acid residues of SEQ ID NO 6.

4. A peptide according to any preceding claim, wherein the immunogenic part of the sequence comprises SEQ ID NO. 9 at the N-terminus and/or SEQ ID NO. 10 at the C-terminus.

5. A peptide according to claim 1, wherein the immunogenic part of the sequence consists of SEQ ID NO 6.

6. A peptide according to any preceding claim, wherein the immune response is stimulated against Colorectal Cancer cells.

7. A peptide according to any of claims 1-5, wherein the immune response is stimulated against Melanoma cells.

8. A peptide according to any preceding claim, wherein the peptide is an oligopeptide.

9. A peptide according to claim 1, wherein the MHC class II molecules are the HLA DRβ1*0402 and/or HLA DRβ1*1301 alleles.

10. A peptide, according to any preceding claim, for use in the method of treatment of colorectal cancer by stimulation of the production of antibodies against said peptide.

11. A peptide according to claim 10, wherein stimulation of the production of antibodies against said peptide is stimulated in the patient in conjunction with PBMC's allogeneic or autologous for at least one sympathetic HLA-II allele capable of presenting all or an immunogenic part of the amino acid sequence designated SEQ ID NO 6 in an immunogenic manner.

12. A peptide according to claim 11, wherein the allele is selected from HLA DRβ1 *0402 and HLA DRβ1*1301.

13. A peptide according to any of claims 10-12, for use in the method of treatment of colorectal cancer in a patient having PBMC's autologous or allogeneic for at least one sympathetic HLA-II allele capable of presenting the COA-1 epitope in an immunogenic manner, wherein in said treatment is by administration of a vaccine comprising the immunising portion of SEQ ID NO 6, or a precursor therefor consisting of a fusion peptide comprising SEQ ID NO 6 or a nucleic acid suitable for expression of SEQ ID NO 6 in PBMC's, to the patient.

14. A peptide for use in the method of treatment of colorectal cancer in a patient,wherein said treatment is by:
- introduction of transformed PBMC's into the patient; and
- administration of a vaccine comprising the immunising portion of SEQ ID NO. 6, or a precursor therefor consisting of a fusion peptide comprising SEQ ID NO 6 or a nucleic acid suitable for expression of SEQ ID NO 6 in PBMC's, to the patient,
the PBMC's, or their progenitors, having been isolated from the patient and transformed with at least one sympathetic HLA-II allele capable of presenting the COA-1 epitope in an immunogenic manner.

15. A peptide according to claim 14, for use in the treatment of colorectal cancer, wherein said treatment is by administration of the immunising portion of SEQ ID NO. 6, or a precursor therefor consisting of a fusion peptide comprising SEQ ID NO 6 or a nucleic acid suitable for expression of SEQ ID NO 6 in PBMC's, with the transformed PBMC's.

16. Use of a peptide according to any preceding claim in the manufacture of a vaccine to stimulate an anti-colorectal cancer or an anti-melanoma immune response against COA-1 (SEQ ID NO 2.

17. A vaccine for use in the method of treatment of colorectal cancer and melanoma, said vaccine comprising a peptide according to any of claims 1-15.

18. A vaccine according to claim 17 comprising a suitable carrier.

19. A vaccine according to any of claims 17-18, comprising the peptide and PBMC's expressing a sympathetic MHC Class II allele therefor.

20. A vaccine according to claim 19, wherein the MHC Class II allele is the HLA DRβ1*0402 and/or the HLA DRβ1*1301 allele.

21. A vaccine according to any of claims 17-20, which further comprises PBMC's (Peripheral Blood Mononuclear Cells) either expressing the HLA DRβ1*0402 and/or the HLA DRβ1*1301 alleles.

## Patentansprüche

1. Peptid, das die gesamte oder einen immunogenen Teil der mit SEQ ID Nr.: 6 bezeichneten Aminosäuresequenz aufweist, zur Verwendung bei einem Verfahren zur Impfstoffbehandlung von Kolorektalkrebs oder Melanom durch Stimulieren einer Anti-Kolorektalkrebs- oder Anti-Melanom-Immunantwort gegen COA-1 (SEQ ID Nr.: 2), wobei der immunogene Teil der Sequenz durch antigenpräsentierende Zellen in Verbindung mit MHC-Molekülen der Klasse II verarbeitet und exprimiert wird.

2. Peptid nach Anspruch 1, wobei der immunogene Teil der Sequenz 8 oder mehr zusammenhängende Aminosäurereste von SEQ ID Nr.: 6 aufweist.

3. Peptid nach Anspruch 2, wobei der immunogene Teil der Sequenz 10 oder mehr zusammenhängende Aminosäurereste von SEQ ID Nr.: 6 aufweist.

4. Peptid nach einem der vorstehenden Ansprüche, wobei der immunogene Teil der Sequenz am N-Terminus SEQ ID Nr.: 9 und/oder am C-Terminus SEQ ID Nr.: 10 aufweist.

5. Peptid nach Anspruch 1, wobei der immunogene Teil der Sequenz aus SEQ ID Nr.: 6 besteht.

6. Peptid nach einem der vorstehenden Ansprüche, wobei die Immunantwort der Sequenz gegen Kolorektalkrebszellen stimuliert wird.

7. Peptid nach einem der Ansprüche 1-5, wobei die Immunantwort gegen Melanomzellen stimuliert wird.

8. Peptid nach einem der vorstehenden Ansprüche, wobei das Peptid ein Oligopeptid ist.

9. Peptid nach Anspruch 1, wobei die MHC-Moleküle der Klasse II die Allele HLA DRβ1*0402 und/oder HLA DRβ1*1301 sind.

10. Peptid nach einem der vorstehenden Ansprüche zur Verwendung bei einem Verfahren zur Behandlung von Kolorektalkrebs durch Stimulieren der Produktion von Antikörpern gegen das Peptid.

11. Peptid nach Anspruch 10, wobei die Produktion von Antikörpern gegen das Peptid in dem Patienten in Verbindung mit mononukleären peripheren Blutzellen (PBMCs) stimuliert wird, die allogen oder autolog für mindestens ein sympathetisches HLA-II-Allel sind, das die gesamte oder einen immunogenen Teil der mit SEQ ID Nr.: 6 bezeichneten Aminosäuresequenz auf immunogene Weise präsentieren kann.

12. Peptid nach Anspruch 11, wobei das Allel unter HLA DRβ1*0402 und HLA DRβ1*1301 ausgewählt ist.

13. Peptid nach einem der Ansprüche 10-12 zur Verwendung bei einem Verfahren zur Behandlung von Kolorektalkrebs bei einem Patienten mit mononukleären peripheren Blutzellen (PBMCs), die allogen oder autolog für mindestens ein sympathetisches HLA-II-Allel sind, welches das COA-1-Epitop auf immunogene Weise präsentieren kann, wobei die Behandlung erfolgt, indem ein Impfstoff mit dem immunisierenden Teil von SEQ ID Nr.: 6 oder einem Vorläufer davon, der aus einem Fusionspeptid mit SEQ ID Nr.: 6 oder einer für die Expression von SEQ ID Nr.: 6 in PBMCs geeigneten Nucleinsäure besteht, an den Patienten verabreicht wird.

14. Peptid zur Verwendung bei einem Verfahren zur Behandlung von Kolorektalkrebs bei einem Patienten, wobei die Behandlung wie folgt ausgeführt wird:
- Einbringen von transformierten PBMCs in den Patienten; und
- Verabreichen eines Impfstoffs mit dem immunisierenden Teil von SEQ ID Nr.: 6 oder einem Vorläufer davon, der aus einem Fusionspeptid mit SEQ ID Nr.: 6 oder einer für die Expression von SEQ ID Nr.: 6 in PBMCs geeigneten Nucleinsäure besteht, an den Patienten,
wobei die PBMCs oder ihre Vorläufer aus dem Patienten isoliert und mit mindestens einem sympathetischen HLA-II-Allel transformiert worden sind, welches das COA-1-Epitop auf immunogene Weise präsentieren kann.

15. Peptid nach Anspruch 14 zur Verwendung bei der Behandlung von Kolorektalkrebs, wobei die Behandlung erfolgt, indem der immunisierende Teil von SEQ ID Nr.: 6 oder ein Vorläufer davon, der aus einem Fusionspeptid mit SEQ ID Nr.: 6 oder einer für die Expression von SEQ ID Nr.: 6 in PBMCs geeigneten Nucleinsäure besteht, zusammen mit den transformierten PBMCs verabreicht wird.

16. Verwendung eines Peptids nach einem der vorstehenden Ansprüche bei der Herstellung eines Impfstoffs zur Stimulierung einer Anti-Kolorektalkrebs- oder einer Anti-Melanom-Immunantwort gegen COA-1 (SEQ ID Nr.: 2).

17. Impfstoff zur Verwendung bei einem Verfahren zur Behandlung von Kolorektalkrebs und Melanom, wobei der Impfstoff ein Peptid nach einem der Ansprüche 1-15 aufweist.

18. Impfstoff nach Anspruch 17, der einen geeigneten Träger aufweist.

19. Impfstoff nach einem der Ansprüche 17-18, der das Peptid und PBMCs aufweist, die ein sympathetisches MHC-Allel der Klasse II dafür exprimieren.

20. Impfstoff nach Anspruch 19, wobei das MHC-Allel der Klasse II das Allel HLA DRβ1*0402 und/oder HLA DRβ1*1301 ist.

21. Impfstoff nach einem der Ansprüche 17-20, der ferner PBMCs (mononukleäre periphere Blutzellen) aufweist, die das Allel HLA DRβ1*0402 und/oder das Allel HLA DRβ1*1301 exprimieren.

## Revendications

1. Peptide, comprenant la totalité ou une partie immunogène de la séquence d'acides aminés désignée SEQ ID NO 6, pour une utilisation dans une méthode de traitement par vaccin d'un cancer colorectal ou d'un mélanome par la stimulation d'une réponse immunitaire anti-cancer colorectal ou anti-mélanome contre COA-1 (SEQ ID NO 2), dans lequel la partie immunogène de la séquence est traitée et exprimée par des cellules présentant un antigène en association avec des molécules de MHC sympathique de classe II.

2. Peptide selon la revendication 1, dans lequel la partie immunogène de la séquence comprend 8 ou plus résidus d'acide aminé contigus de la SEQ ID NO 6.

3. Peptide selon la revendication 2, dans lequel la partie immunogène de la séquence comprend 10 ou plus résidus d'acide aminé contigus de la SEQ ID NO 6.

4. Peptide selon l'une quelconque des revendications précédentes, dans lequel la partie immunogène de la séquence comprend la SEQ ID NO 9 à la terminaison N et/ou la SEQ ID NO 10 à la terminaison C.

5. Peptide selon la revendication 1, dans lequel la partie immunogène de la séquence est constitué de la SEQ ID NO 6.

6. Peptide selon l'une quelconque des revendications précédentes, dans lequel la réponse immunitaire est stimulée contre des cellules de cancer colorectal.

7. Peptide selon l'une quelconque des revendications 1-5, dans lequel la réponse immunitaire est stimulée contre des cellules de mélanome.

8. Peptide selon l'une quelconque des revendications précédentes, où le peptide est un oligopeptide.

9. Peptide selon la revendication 1, dans lequel les molécules de MHC de classe II sont les allèles HLA DRβ1*0402 et/ou HLA DRβ1*1301.

10. Peptide selon l'une quelconque des revendications précédentes, pour une utilisation dans la méthode de traitement d'un cancer colorectal par la stimulation de la production d'anticorps contre ledit peptide.

11. Peptide selon la revendication 10, dans lequel la stimulation de la production d'anticorps contre ledit peptide est effectuée chez le patient conjointement à des PBMC allogéniques ou autologues pour au moins un allèle HLA-II sympathique capable de présenter la totalité ou une partie immunogène de la séquence d'acides aminés désignée SEQ ID NO 6 d'une manière immunogène.

12. Peptide selon la revendication 11, dans lequel l'allèle est choisi parmi HLA DRβ1*0402 et HLA DRβ1*1301.

13. Peptide selon l'une quelconque des revendications 10-12, pour une utilisation dans la méthode de traitement d'un cancer colorectal chez un patient possédant des PBMC autologues ou allogéniques pour au moins un allèle HLA-II sympathique capable de présenter l'épitope COA-1 d'une manière immunogène, dans lequel ledit traitement se fait par l'administration d'un vaccin comprenant la portion immunisante de la SEQ ID NO 6, ou un précurseur pour celle-ci constitué d'un peptide de fusion comprenant la SEQ ID NO 6 ou un acide nucléique approprié pour une expression de la SEQ ID NO 6 dans les PBMC, au patient.

14. Peptide pour une utilisation dans la méthode de traitement d'un cancer de colorectal chez un patient, dans lequel ledit traitement se fait par:
- l'introduction de PBMC transformées dans le patient; et
- l'administration d'un vaccin comprenant la portion immunisante de la SEQ ID NO 6, ou un précurseur pour celle-ci constitué d'un peptide de fusion comprenant la SEQ ID NO 6 ou un acide nucléique approprié pour une expression de la SEQ ID NO 6 dans les PBMC, au patient,
les PBMC, ou leurs précurseurs, ayant été isolées à partir du patient et transformées avec au moins un allèle HLA-II sympathique capable de présenter l'épitope COA-1 d'une manière immunogène.

15. Peptide selon la revendication 14, pour une utilisation dans le traitement d'un cancer colorectal, dans lequel ledit traitement se fait par l'administration de la portion immunisante de la SEQ ID NO 6, ou un précurseur pour celle-ci constitué d'une protéine de fusion comprenant la SEQ ID NO 6 ou un acide nucléique approprié pour une expression de la SEQ ID NO 6 dans des PBMC, avec les PBMC transformées.

16. Utilisation d'un peptide selon l'une quelconque des revendications précédentes dans la fabrication d'un vaccin pour stimuler une réponse immunitaire anti-cancer colorectal ou anti-mélanome contre COA-1 (SEQ ID NO 2).

17. Vaccin pour une utilisation dans la méthode de traitement d'un cancer colorectal et d'un mélanome, ledit vaccin comprenant un peptide selon l'une quelconque des revendications 1-15.

18. Vaccin selon la revendication 17, comprenant un support approprié.

19. Vaccin selon l'une quelconque des revendications 17-18, comprenant le peptide et des PBMC exprimant un allèle de MHC sympathique de classe II pour celui-ci.

20. Vaccin selon la revendication 19, dans lequel l'allèle de MHC de classe II est l'allèle HLA DRβ1 *0402 et/ou HLA DRβ1 *1301.

21. Vaccin selon l'une quelconque des revendications 17-20, qui comprend en outre des PBMC (cellules mononucléaires de sang périphérique) exprimant les allèles HLA DRβ1*0402 et/ou HLA DRβ1*1301.


### SEQUENCE LISTING

<110> Istituto Superiore di Sanità
   National Institutes of Health
<120> COLORECTAL ANTIGEN
<130> WPP88367
<150> US 60/512,040
   <151> 2003-10-15
<160> 20
<170> PatentIn version 3.3
<210> 1
   <211> 1413
   <212> DNA
   <213> Homo sapiens
<220>
   <221> CDS
   <222> (85)..(1395)
<220>
   <221> misc_feature
   <222> (1180)..(1240)
   <223> nucleotide sequence encoding the immunogenic peptide
<400> 1
   cgctgcggga cggctagcgg ccctgcgtgg aggcgaggaa tccgcatcta tggagatgtc     60
   cctgcatccc atgactcgga gctg atg gcc ttc atg acg agg aag ttg tgg       111
                              Met Ala Phe Met Thr Arg Lys Leu Trp
                              1               5
   gac ctg gag cag cag gtg aag gcc cag act gat gag ata ctg tcc aag      159
   Asp Leu Glu Gln Gln Val Lys Ala Gln Thr Asp Glu Ile Leu Ser Lys
   10                  15                  20                  25
   gat cag aag ata gcg gcc cta gag gac ctg gtg cag acc ctc cgg cca      207
   Asp Gln Lys Ile Ala Ala Leu Glu Asp Leu Val Gln Thr Leu Arg Pro
                   30                  35                  40
   cac cca gcc gag gca acc ctg cag cgg cag gag gaa ctg gag acg atg      255
   His Pro Ala Glu Ala Thr Leu Gln Arg Gln Glu Glu Leu Glu Thr Met
               45                  50                  55
   tgt gtg cag ctg cag cgg cag gtc agg gag atg gag cgg ttc ctc agt      303
   Cys Val Gln Leu Gln Arg Gln Val Arg Glu Met Glu Arg Phe Leu Ser
           60                  65                  70
   gac tat ggc ctg cag tgg gtg ggc gag ccc atg gac cag gag gac tca      351
   Asp Tyr Gly Leu Gln Trp Val Gly Glu Pro Met Asp Gln Glu Asp Ser
       75                  80                  85
   gag agc aag aca gtc tca gag cat ggc gag agg gac tgg atg aca gcc      399
   Glu Ser Lys Thr Val Ser Glu His Gly Glu Arg Asp Trp Met Thr Ala
   90                  95                  100                 105
   aag aag ttc tgg aag cca ggg gac tca ttg gcg ccc cct gag gtg gac      447
   Lys Lys Phe Trp Lys Pro Gly Asp Ser Leu Ala Pro Pro Glu Val Asp
                   110                 115                 120
   ttt gac agg ctg ctg gcc agc ctg cag gat ctt agt gag ctg gtg gta      495
   Phe Asp Arg Leu Leu Ala Ser Leu Gln Asp Leu Ser Glu Leu Val Val
               125                 130                 135
   gag ggt gac acc caa gtg aca cca gtg ccc ggc ggg gca cgg ctg cgt       543
   Glu Gly Asp Thr Gln Val Thr Pro Val Pro Gly Gly Ala Arg Leu Arg
           140                 145                 150
   acc ctc gag ccc atc ccg ctg aag ctc tac cgg aat ggc atc atg atg       591
   Thr Leu Glu Pro Ile Pro Leu Lys Leu Tyr Arg Asn Gly Ile Met Met
       155                 160                 165
   ttc gac ggg ccc ttc cag ccc ttc tac gat ccc tcc aca cag cgc tgc       639
   Phe Asp Gly Pro Phe Gln Pro Phe Tyr Asp Pro Ser Thr Gln Arg Cys
   170                 175                 180                 185
   ctc cga gac ata ttg gat ggc ttc ttt ccc tca gag ctc cag cga ctg       687
   Leu Arg Asp Ile Leu Asp Gly Phe Phe Pro Ser Glu Leu Gln Arg Leu
                   190                 195                 200
   tac ccc aat ggg gtc ccc ttt aag gtg agt gac ttg cgc aat cag gtc       735
   Tyr Pro Asn Gly Val Pro Phe Lys Val Ser Asp Leu Arg Asn Gln Val
               205                 210                 215
   tac ctg gag gat gga ctg gac ccc ttc cca ggc gag ggc cgt gtg gtg       783
   Tyr Leu Glu Asp Gly Leu Asp Pro Phe Pro Gly Glu Gly Arg Val Val
           220                 225                 230
   ggc agg cag cgg atg cac aag gcc ttg gac agg gtg gag gag cac cca       831
   Gly Arg Gln Arg Met His Lys Ala Leu Asp Arg Val Glu Glu His Pro
       235                 240                 245
   ggc tcc agg atg act gct gag aaa ttt ctg aac agg ctc ccc aag ttt       879
   Gly Ser Arg Met Thr Ala Glu Lys Phe Leu Asn Arg Leu Pro Lys Phe
   250                 255                 260                 265
   gtg atc cgg caa ggc gag gtg att gac atc cgg ggc ccc atc agg gac       927
   Val Ile Arg Gln Gly Glu Val Ile Asp Ile Arg Gly Pro Ile Arg Asp
                   270                 275                 280
   acc ttg cag aac tgc tgc cca ttg cct gcc cgg atc cag gag att gtg       975
   Thr Leu Gln Asn Cys Cys Pro Leu Pro Ala Arg Ile Gln Glu Ile Val
               285                 290                 295
   gtg gag acg ccc acc ttg gcc gct gag cga gag agg agc cag gag tca      1023
   Val Glu Thr Pro Thr Leu Ala Ala Glu Arg Glu Arg Ser Gln Glu Ser
           300                 305                 310
   ccc aac aca ccg gca ccc ccg ctc tcc atg ctg cgc atc aag tct gag      1071
   Pro Asn Thr Pro Ala Pro Pro Leu Ser Met Leu Arg Ile Lys Ser Glu
       315                 320                 325
   aat ggg gaa cag gcc ttc cta ctg atg atg cag cct gac aac acc att      1119
   Asn Gly Glu Gln Ala Phe Leu Leu Met Met Gln Pro Asp Asn Thr Ile
   330                 335                 340                 345
   ggg gac gtg cga gct ctg cta gcg cag gcc agg gtc atg gat gcc tct      1167
   Gly Asp Val Arg Ala Leu Leu Ala Gln Ala Arg Val Met Asp Ala Ser
                   350                 355                 360
   gcc ttt gag atc ttc agc aca ttc ccg ccc acc ctc tac cag gac gat      1215
   Ala Phe Glu Ile Phe Ser Thr Phe Pro Pro Thr Leu Tyr Gln Asp Asp
               365                 370                 375
   aca ctc acg ctg cag gct gca ggc ctt gtg ccc aaa gca gca ctg ctg      1263
   Thr Leu Thr Leu Gln Ala Ala Gly Leu Val Pro Lys Ala Ala Leu Leu
           380                 385                 390
   ctg cgg gca cgc cga gcc ccg aag tcc agc ctg aaa ttc agt cct ggt      1311
   Leu Arg Ala Arg Arg Ala Pro Lys Ser Ser Leu Lys Phe Ser Pro Gly
       395                 400                 405
   ccc tgt ccc ggt ccc ggt ccc ggc ccc agt ccc ggt ccc ggt ccc ggc      1359
   Pro Cys Pro Gly Pro Gly Pro Gly Pro Ser Pro Gly Pro Gly Pro Gly
   410                 415                 420                 425
   tcc agt ccc tgt ccc gga ccc agt ccc agc ccc caa taaagcaccc           1405
   Ser Ser Pro Cys Pro Gly Pro Ser Pro Ser Pro Gln
                   430                 435
   accccctc                                                             1413
<210> 2
   <211> 437
   <212> PRT
   <213> Homo sapiens
<400> 2
   Met Ala Phe Met Thr Arg Lys Leu Trp Asp Leu Glu Gln Gln Val Lys
   1               5                   10                  15
   Ala Gln Thr Asp Glu Ile Leu Ser Lys Asp Gln Lys Ile Ala Ala Leu
               20                  25                  30
   Glu Asp Leu Val Gln Thr Leu Arg Pro His Pro Ala Glu Ala Thr Leu
           35                  40                  45
   Gln Arg Gln Glu Glu Leu Glu Thr Met Cys Val Gln Leu Gln Arg Gln
       50                  55                  60
   Val Arg Glu Met Glu Arg Phe Leu Ser Asp Tyr Gly Leu Gln Trp Val
   65                  70                  75                  80
   Gly Glu Pro Met Asp Gln Glu Asp Ser Glu Ser Lys Thr Val Ser Glu
                   85                  90                  95
   His Gly Glu Arg Asp Trp Met Thr Ala Lys Lys Phe Trp Lys Pro Gly
               100                 105                 110
   Asp Ser Leu Ala Pro Pro Glu Val Asp Phe Asp Arg Leu Leu Ala Ser
           115                 120                 125
   Leu Gln Asp Leu Ser Glu Leu Val Val Glu Gly Asp Thr Gln Val Thr
       130                 135                 140
   Pro Val Pro Gly Gly Ala Arg Leu Arg Thr Leu Glu Pro Ile Pro Leu
   145                 150                 155                 160
   Lys Leu Tyr Arg Asn Gly Ile Met Met Phe Asp Gly Pro Phe Gln Pro
                   165                 170                 175
   Phe Tyr Asp Pro Ser Thr Gln Arg Cys Leu Arg Asp Ile Leu Asp Gly
               180                 185                 190
   Phe Phe Pro Ser Glu Leu Gln Arg Leu Tyr Pro Asn Gly Val Pro Phe
   195 200 205
   Lys Val Ser Asp Leu Arg Asn Gln Val Tyr Leu Glu Asp Gly Leu Asp
   210 215 220
   Pro Phe Pro Gly Glu Gly Arg Val Val Gly Arg Gln Arg Met His Lys
   225 230 235 240
   Ala Leu Asp Arg Val Glu Glu His Pro Gly Ser Arg Met Thr Ala Glu
   245 250 255
   Lys Phe Leu Asn Arg Leu Pro Lys Phe Val Ile Arg Gln Gly Glu Val
   260 265 270
   Ile Asp Ile Arg Gly Pro Ile Arg Asp Thr Leu Gln Asn Cys Cys Pro
   275 280 285
   Leu Pro Ala Arg Ile Gln Glu Ile Val Val Glu Thr Pro Thr Leu Ala
   290 295 300
   Ala Glu Arg Glu Arg Ser Gln Glu Ser Pro Asn Thr Pro Ala Pro Pro
   305 310 315 320
   Leu Ser Met Leu Arg Ile Lys Ser Glu Asn Gly Glu Gln Ala Phe Leu
   325 330 335
   Leu Met Met Gln Pro Asp Asn Thr Ile Gly Asp Val Arg Ala Leu Leu
   340 345 350
   Ala Gln Ala Arg Val Met Asp Ala Ser Ala Phe Glu Ile Phe Ser Thr
   355 360 365
   Phe Pro Pro Thr Leu Tyr Gln Asp Asp Thr Leu Thr Leu Gln Ala Ala
   370 375 380
   Gly Leu Val Pro Lys Ala Ala Leu Leu Leu Arg Ala Arg Arg Ala Pro
   385 390 395 400
   Lys Ser Ser Leu Lys Phe Ser Pro Gly Pro Cys Pro Gly Pro Gly Pro
   405 410 415
   Gly Pro Ser Pro Gly Pro Gly Pro Gly Ser Ser Pro Cys Pro Gly Pro
   420 425 430
   Ser Pro Ser Pro Gln
   435
<210> 3
   <211> 60
   <212> DNA
   <213> Homo sapiens
<400> 3
   ttcagcacat tcccgcccac cctctaccag gacgatacac tcacgctgca ggctgcaggc 60
<210> 4
   <211> 20
   <212> PRT
   <213> Homo sapiens
<400> 4
   Phe Ser Thr Phe Pro Pro Thr Leu Tyr Gln Asp Asp Thr Leu Thr Leu
   1               5                   10                  15
   Gln Ala Ala Gly
               20
<210> 5
   <211> 42
   <212> DNA
   <213> Homo sapiens
<400> 5
   accctctacc aggacgatac actcacgctg caggctgcag gc 42
<210> 6
   <211> 14
   <212> PRT
   <213> Homo sapiens
<400> 6
   Thr Leu Tyr Gln Asp Asp Thr Leu Thr Leu Gln Ala Ala Gly
   1               5                   10
<210> 7
   <211> 1028
   <212> DNA
   <213> Homo sapiens
<400> 7
   ctcagtgact atggcctgca gtgggtgggc gagcccatgg accaggagga ctcagagagc      60
   aagacagtct cagagcatgg cgagagggac tggatgacag ccaagaagtt ctggaagcca     120
   ggggactcat tggcgccccc tgaggtggac tttgacaggc tgctggccag cctgcaggat     180
   cttagtgagc tggtggtaga gggtgacacc caagtgacac cagtgcccgg cggggcacgg     240
   ctgcgtaccc tcgagcccat cccgctgaag ctctaccgga atggcatcat gatgttcgac     300
   gggcccttcc agcccttcta cgatccctcc acacagcgct gcctccgaga catattggat     360
   ggcttctttc cctcagagct ccagcgactg taccccaatg gggtcccctt taaggtgagt     420
   gacttgcgca atcaggtcta cctggaggat ggactggacc ccttcccagg cgagggccgt     480
   gtggtgggca ggcagcggat gcacaaggcc ttggacaggg tggaggagca cccaggctcc     540
   aggatgactg ctgagaaatt tctgaacagg ctccccaagt tttgatccgg caaggcgagg     600
   tgattgacat ccggggcccc atcagggaca ccttgcagaa ctgctgccca ttgcctgccc     660
   ggatccagga gattgtggtg gagacgccca ccttggccgc tgagcgagag aggagccagg     720
   agtcacccaa cacaccggca cccccgctct ccatgctgcg catcaagtct gagaatgggg     780
   aacaggcctt cctactgatg atgcagcctg acaacaccat tggggacgtg cgagctctgc     840
   tagcgcaggc cagggtcatg gatgcctctg cctttgagat cttcagcaca ttcccgccca     900
   ccctctacca ggacgataca ctcacgctgc aggctgcagg ccttgtgccc aaagcagcac     960
   tgctgctgcg ggcacgccga gccccgaagt ccagcctgaa attcagtcct ggtccctgtc    1020
   ccggtccc                                                             1028
<210> 8
   <211> 343
   <212> PRT
   <213> Homo sapiens
<400> 8
   Leu Ser Asp Tyr Gly Leu Gln Trp Val Gly Glu Pro Met Asp Gln Glu
   1               5                   10                  15
   Asp Ser Glu Ser Lys Thr Val Ser Glu His Gly Glu Arg Asp Trp Met
               20                  25                  30
   Thr Ala Lys Lys Phe Trp Lys Pro Gly Asp Ser Leu Ala Pro Pro Glu
           35                  40                  45
   Val Asp Phe Asp Arg Leu Leu Ala Ser Leu Gln Asp Leu Ser Glu Leu
       50                  55                  60
   Val Val Glu Gly Asp Thr Gln Val Thr Pro Val Pro Gly Gly Ala Arg
   65                  70                  75                  80
   Leu Arg Thr Leu Glu Pro Ile Pro Leu Lys Leu Tyr Arg Asn Gly Ile
                   85                  90                  95
   Met Met Phe Asp Gly Pro Phe Gln Pro Phe Tyr Asp Pro Ser Thr Gln
               100                 105                 110
   Arg Cys Leu Arg Asp Ile Leu Asp Gly Phe Phe Pro Ser Glu Leu Gln
           115                 120                 125
   Arg Leu Tyr Pro Asn Gly Val Pro Phe Lys Val Ser Asp Leu Arg Asn
       130                 135                 140
   Gln Val Tyr Leu Glu Asp Gly Leu Asp Pro Phe Pro Gly Glu Gly Arg
   145                 150                 155                 160
   Val Val Gly Arg Gln Arg Met His Lys Ala Leu Asp Arg Val Glu Glu
                   165                 170                 175
   His Pro Gly Ser Arg Met Thr Ala Glu Lys Phe Leu Asn Arg Leu Pro
               180                 185                 190
   Lys Phe Val Ile Arg Gln Gly Glu Val Ile Asp Ile Arg Gly Pro Ile
   195 200 205
   Arg Asp Thr Leu Gln Asn Cys Cys Pro Leu Pro Ala Arg Ile Gln Glu
   210 215 220
   Ile Val Val Glu Thr Pro Thr Leu Ala Ala Glu Arg Glu Arg Ser Gln
   225 230 235 240
   Glu Ser Pro Asn Thr Pro Ala Pro Pro Leu Ser Met Leu Arg Ile Lys
   245 250 255
   Ser Glu Asn Gly Glu Gln Ala Phe Leu Leu Met Met Gln Pro Asp Asn
   260 265 270
   Thr Ile Gly Asp Val Arg Ala Leu Leu Ala Gln Ala Arg Val Met Asp
   275 280 285
   Ala Ser Ala Phe Glu Ile Phe Ser Thr Phe Pro Pro Thr Leu Tyr Gln
   290 295 300
   Asp Asp Thr Leu Thr Leu Gln Ala Ala Gly Leu Val Pro Lys Ala Ala
   305 310 315 320
   Leu Leu Leu Arg Ala Arg Arg Ala Pro Lys Ser Ser Leu Lys Phe Ser
   325 330 335
   Pro Gly Pro Cys Pro Gly Pro
   340
<210> 9
   <211> 6
   <212> PRT
   <213> Homo sapiens
<400> 9
   Phe Ser Thr Phe Pro Pro
   1 5
<210> 10
   <211> 6
   <212> PRT
   <213> Homo sapiens
<400> 10
   Leu Val Pro Lys Ala Ala
   1 5
<210> 11
   <211> 294
   <212> DNA
   <213> Homo sapiens
<400> 11
   ggggacgtgc gagctctgct agcgcaggcc agggtcatgg atgcctctgc ctttgagatc      60
   ttcagcacat tcccgcccac cctctaccag gacgatacac tcacgctgca ggctgcaggc     120
   cttgtgccca aagcagcact gctgctgcgg gcacgccgag ccccgaagtc cagcctgaaa     180
   ttcagtcctg gtccctgtcc cggtcccggt cccggcccca gtcccggtcc cggtcccggc     240
   tccagtccct gtcccggacc cagtcccagc ccccaataaa gcacccaccc cctc           294
<210> 12
   <211> 92
   <212> PRT
   <213> Homo sapiens
<400> 12
   Gly Asp Val Arg Ala Leu Leu Ala Gln Ala Arg Val Met Asp Ala Ser
   1               5                   10                  15
   Ala Phe Glu Ile Phe Ser Thr Phe Pro Pro Thr Leu Tyr Gln Asp Asp
               20                  25                  30
   Thr Leu Thr Leu Gln Ala Ala Gly Leu Val Pro Lys Ala Ala Leu Leu
           35                  40                  45
   Leu Arg Ala Arg Arg Ala Pro Lys Ser Ser Leu Lys Phe Ser Pro Gly
       50                  55                  60
   Pro Cys Pro Gly Pro Gly Pro Gly Pro Ser Pro Gly Pro Gly Pro Gly
   65                  70                  75                  80
   Ser Ser Pro Cys Pro Gly Pro Ser Pro Ser Pro Gln
                   85                  90
<210> 13
   <211> 19
   <212> DNA
   <213> ARTIFICIAL
<220>
   <223> PCR primer sequence
<400> 13
   tccagcatgg tgtgtctga 19
<210> 14
   <211> 18
   <212> DNA
   <213> artificial
<220>
   <223> PCR primer sequence
<400> 14
   ccttgaatgt ggtcatct 18
<210> 15
   <211> 23
   <212> DNA
   <213> artificial
<220>
   <223> PCR primer sequence
<400> 15
   cgtttcttgg agtactctac gtc 23
<210> 16
   <211> 20
   <212> DNA
   <213> artificial
<220>
   <223> PCR primer sequence
<400> 16
   ccaccgcggc ccgctcgtct 20
<210> 17
   <211> 20
   <212> PRT
   <213> Homo sapiens
<400> 17
   Phe Ser Thr Phe Pro Pro Thr Leu Tyr Gln Asp Asp Thr Leu Thr Leu
   1               5                   10                  15
   Gln Ala Ala Gly
               20
<210> 18
   <211> 20
   <212> PRT
   <213> Homo sapiens
<400> 18
   Thr Leu Tyr Gln Asp Asp Thr Leu Thr Leu Gln Ala Ala Gly Leu Val
   1               5                   10                  15
   Pro Lys Ala Ala
               20
<210> 19
   <211> 1771
   <212> DNA
   <213> Homo sapiens
<400> 19
   aaaaaaccgc gtgacaacaa gatggcggcg ctgcgggacg gctagcggcc ctgcgtgtac      60
   tttcccaagc accaccaggc caaaggtctc tcagttcaga gcagaaagcc gtatacccag     120
   aggagcaggc agataacaga aacttccaga aacctctgtg gagacagtgg aagaggcaaa     180
   agggagttcc tgacagctgg attctagaag tagaactatg agctcacctt tggcctccct     240
   tagcaagacc cgaaaagtgc ccctgccctc ggagcctatg aatcctggga ggcgaggaat    300
   ccgcatctat ggagatgaag atgaggtgga catgttgagt gatgggtgtg gctcggaaga    360
   aaagatctca gtcccttcct gctatggcgg cataggtgcc cctgtgagtc ggcaagtccc    420
   tgcatcccat gactcggagc tgatggcctt catgacgagg aagttgtggg acctggagca    480
   gcaggtgaag gcccagactg atgagatact gtccaaggat cagaagatag cggccctaga    540
   ggacctggtg cagaccctcc ggccacaccc agccgaggca accctgcagc ggcaggagga    600
   actggagacg atgtgtgtgc agctgcagcg gcaggtcagg gagatggagc ggttcctcag    660
   tgactatggc ctgcagtggg tgggcgagcc catggaccag gaggactcag agagcaagac    720
   agtctcagag catggcgaga gggactggat gacagccaag aagttctgga agccagggga    780
   ctcattggcg ccccctgagg tggactttga caggctgctg gccagcctgc aggatcttag    840
   tgagctggtg gtagagggtg acacccaagt gacaccagtg cccggcgggg cacggctgcg    900
   taccctcgag cccatcccgc tgaagctcta ccggaatggc atcatgatgt tcgacgggcc    960
   cttccagccc ttctacgatc cctccacaca gcgctgcctc cgagacatat tggatggctt   1020
   ctttccctca gagctccagc gactgtaccc caatggggtc ccctttaagg tgagtgactt   1080
   gcgcaatcag gtctacctgg aggatggact ggaccccttc ccaggcgagg gccgtgtggt   1140
   gggcaggcag cggatgcaca aggccttgga cagggtggag gagcacccag gctccaggat   1200
   gactgctgag aaatttctga acaggctccc caagtttgtg atccggcaag gcgaggtgat   1260
   tgacatccgg ggccccatca gggacacctt gcagaactgc tgcccattgc ctgcccggat   1320
   ccaggagatt gtggtggaga cgcccacctt ggccgctgag cgagagagga gccaggagtc   1380
   acccaacaca ccggcacccc cgctctccat gctgcgcatc aagtctgaga atggggaaca   1440
   ggccttccta ctgatgatgc agcctgacaa caccattggg gacgtgcgag ctctgctagc   1500
   gcaggccagg gtcatggatg cctctgcctt tgagatcttc agcacattcc cgcccaccct   1560
   ctaccaggac gatacactca cgctgcaggc tgcaggcctt gtgcccaaag cagcactgct   1620
   gctgcgggca cgccgagccc cgaagtccag cctgaaattc agtcctggtc cctgtcccgg   1680
   tcccggtccc ggccccagtc ccggtcccgg tcccggctcc agtccctgtc ccggacccag   1740
   tcccagcccc caataaagca cccgccccct c                                  1771
<210> 20
   <211> 512
   <212> PRT
   <213> Homo sapiens
<400> 20
   Met Ser Ser Pro Leu Ala Ser Leu Ser Lys Thr Arg Lys Val Pro Leu
   1 5 10 15
   Pro Ser Glu Pro Met Asn Pro Gly Arg Arg Gly Ile Arg Ile Tyr Gly
   20 25 30
   Asp Glu Asp Glu Val Asp Met Leu Ser Asp Gly Cys Gly Ser Glu Glu
   35 40 45
   Lys Ile Ser Val Pro Ser Cys Tyr Gly Gly Ile Gly Ala Pro Val Ser
   50 55 60
   Arg Gln Val Pro Ala Ser His Asp Ser Glu Leu Met Ala Phe Met Thr
   65 70 75 80
   Arg Lys Leu Trp Asp Leu Glu Gln Gln Val Lys Ala Gln Thr Asp Glu
   85 90 95
   Ile Leu Ser Lys Asp Gln Lys Ile Ala Ala Leu Glu Asp Leu Val Gln
   100 105 110
   Thr Leu Arg Pro His Pro Ala Glu Ala Thr Leu Gln Arg Gln Glu Glu
   115 120 125
   Leu Glu Thr Met Cys Val Gln Leu Gln Arg Gln Val Arg Glu Met Glu
   130 135 140
   Arg Phe Leu Ser Asp Tyr Gly Leu Gln Trp Val Gly Glu Pro Met Asp
   145 150 155 160
   Gln Glu Asp Ser Glu Ser Lys Thr Val Ser Glu His Gly Glu Arg Asp
   165 170 175
   Trp Met Thr Ala Lys Lys Phe Trp Lys Pro Gly Asp Ser Leu Ala Pro
   180 185 190
   Pro Glu Val Asp Phe Asp Arg Leu Leu Ala Ser Leu Gln Asp Leu Ser
   195 200 205
   Glu Leu Val Val Glu Gly Asp Thr Gln Val Thr Pro Val Pro Gly Gly
   210 215 220
   Ala Arg Leu Arg Thr Leu Glu Pro Ile Pro Leu Lys Leu Tyr Arg Asn
   225 230 235 240
   Gly Ile Met Met Phe Asp Gly Pro Phe Gln Pro Phe Tyr Asp Pro Ser
   245 250 255
   Thr Gln Arg Cys Leu Arg Asp Ile Leu Asp Gly Phe Phe Pro Ser Glu
   260 265 270
   Leu Gln Arg Leu Tyr Pro Asn Gly Val Pro Phe Lys Val Ser Asp Leu
   275 280 285
   Arg Asn Gln Val Tyr Leu Glu Asp Gly Leu Asp Pro Phe Pro Gly Glu
   290 295 300
   Gly Arg Val Val Gly Arg Gln Arg Met His Lys Ala Leu Asp Arg Val
   305                 310                 315                 320
   Glu Glu His Pro Gly Ser Arg Met Thr Ala Glu Lys Phe Leu Asn Arg
                   325                 330                 335
   Leu Pro Lys Phe Val Ile Arg Gln Gly Glu Val Ile Asp Ile Arg Gly
               340                 345                 350
   Pro Ile Arg Asp Thr Leu Gln Asn Cys Cys Pro Leu Pro Ala Arg Ile
           355                 360                 365
   Gln Glu Ile Val Val Glu Thr Pro Thr Leu Ala Ala Glu Arg Glu Arg
       370                 375                 380
   Ser Gln Glu Ser Pro Asn Thr Pro Ala Pro Pro Leu Ser Met Leu Arg
   385                 390                 395                 400
   Ile Lys Ser Glu Asn Gly Glu Gln Ala Phe Leu Leu Met Met Gln Pro
                   405                 410                 415
   Asp Asn Thr Ile Gly Asp Val Arg Ala Leu Leu Ala Gln Ala Arg Val
               420                 425                 430
   Met Asp Ala Ser Ala Phe Glu Ile Phe Ser Thr Phe Pro Pro Thr Leu
           435                 440                 445
   Tyr Gln Asp Asp Thr Leu Thr Leu Gln Ala Ala Gly Leu Val Pro Lys
       450                 455                 460
   Ala Ala Leu Leu Leu Arg Ala Arg Arg Ala Pro Lys Ser Ser Leu Lys
   465                 470                 475                 480
   Phe Ser Pro Gly Pro Cys Pro Gly Pro Gly Pro Gly Pro Ser Pro Gly
                   485                 490                 495
   Pro Gly Pro Gly Ser Ser Pro Cys Pro Gly Pro Ser Pro Ser Pro Gln
               500                 505                 510